# EUROPÄISCHE PATENTSCHRIFT

(11) **EP 1 688 125 B1**
(45) Veröffentlichungstag und Bekanntmachung des Hinweises auf die Patenterteilung: **29.12.2010**
(21) Anmeldenummer: 06001262.2
(22) Anmeldetag: 20.01.2006
(51) Int. Cl.: A61K 6/083, C07C 69/753, C07C 69/757

(54) **Polymerisierbare Cyclopropylacrylate enthaltende Zusammensetzung**
Composition comprising polymerisable cyclopropylacrylates
Composition comprenant des cyclopropylacrylates polymérisables

(30) Priorität: 07.02.2005 DE 102005005541
(43) Veröffentlichungstag der Anmeldung: 09.08.2006
(73) Patentinhaber: Ivoclar Vivadent AG, 9494 Schaan (LI)
(72) Erfinder: de Meijere, Armin, Prof. Dr., 37077 Göttingen (DE); Moszner, Norbert, Prof. Dr., 9493 Mauren (LI); Bahutski, Viktor, Dr., 37075 Göttingen (DE); Zeuner, Frank, Dr., 9488 Schellenberg (LI); Fischer, Urs-Karl, 9320 Arbon (CH); Rheinberger, Volker, Dr., 9490 Vaduz (LI)
(74) Vertreter: UEXKÜLL & STOLBERG

(56) Entgegenhaltungen:
- DE-A1- 10 249 324
- W. OPPOLZER, A. PIMM ET AL.: "Palladium-Catalysed Intramolecular Cyclisation of Olefinic Propargylic Carbonates and Application to the Diastereoselective Synthesis of Enantiomerically Pure (-)-alpha-Thujone" HELVETICA CHIMICA ACTA., Bd. 80, 1997, Seiten 623-639, XP002380016 CHVERLAG HELVETICA CHIMICA ACTA. BASEL.
- ARMIN DE MEIJERE ET AL.: "Synthesis and Radical Polymerisation of Various 2-Cyclopropylacrylates" EUROPEAN JOURNAL OF ORGANIC CHEMISTRY., Nr. 17, 2004, Seiten 3669-3678, XP002380017 WILEY-VCH VERLAG, WEINHEIM., DE
- MOSZNER, NORBERT ET AL: "Polymerization of cyclic monomers. 12. Radical polymerization of substituted methyl 2-(bicyclo[3.1.0]hex-1-yl)acrylates and properties of thereof based light-curing composites" MACROMOLECULAR MATERIALS AND ENGINEERING, Bd. 291, Nr. 1, 5. Januar 2006 (2006-01-05), Seiten 83-89, XP002380018

## Beschreibung

Die vorliegende Erfindung betrifft polymerisationsfähige Cyclopropylacrylate, die sich insbesondere zur Herstellung von Dentalmaterialien eignen.

Radikalisch polymerisierbare cyclische Monomere sind aufgrund des im Vergleich zu linearen Monomeren deutlich geringeren Polymerisationsschrumpfes von besonderem Interesse (R. K. Sadhir, R. M. Luck, Expanding Monomers, CRC Press, Boca Raton etc. 1992).

Im Gegensatz zu anderen bekannten ringöffnenden Monomeren, wie methylengruppenhaltigen Spiroorthocarbonaten (SOC), Spiroorthoestern (SOE) oder bicyclischen Orthoestern (BOE) sind Vinylcyclopropane nicht feuchtigkeitsempfindlich und ihre radikalische Polymerisation zeichnet sich auch dadurch aus, daß Polymere mit einer relativ hohen Molmasse erhalten werden (N. Moszner, F. Zeuner, T. Völkel, V. Rheinberger, Macromol. Chem. Phys. 200 (1999) 2173). Bei der radikalischen Polymerisation von SOC oder SOE werden Polymere mit Carbonat-Ether- oder Ester-Ether-Gruppen in der Hauptkette gebildet, die dementsprechend hydrolytisch oder enzymatisch einfach spaltbar sind. Demgegenüber führt die Ringöffnungspolymerisation von 1,1-disubstituierten 2-Vinylcyclopropanen zu Polymeren, die in der Hauptkette nur hydrolytisch stabile C-C-Bindungen enthalten.

Die EP 0 798 286 B1 offenbart Monomere mit mehreren Vinylcyclopropylgruppen, die zu unlöslichen Polymernetzwerken führen.

Sanda et al. konnten am Beispiel der radikalischen Copolymerisation von 1,1-Bis(ethoxycarbonyl)-2-vinylcyclopropan mit Methylmethacrylat (MMA) zeigen, daß sich Vinylcyclopropane im Vergleich zu Methacrylaten durch ein geringeres radikalisches Polymerisationsvermögen auszeichnen, was deren praktischen Einsatz deutlich einschränkt (F. Sanda, T. Takata, T. Endo, Macromolecules, 27 (1994) 3982).

Eine verbesserte Reaktivität in der radikalischen Polymerisation zeigen bicyclische Cyclopropylacrylate, wie z.B. 2-[Bi-cyclo[3.1.0]hex-1-yl]-acrylsäuremethylester (N. Moszner, F. Zeuner, U.K. Fischer, V. Rheinberger, A. de Meijere, V. Bagutski, Macromol. Rapid. Commun. 24 (2003) 269), deren Verwendung in Dentalmaterialien in der DE 102 49 324 A1 vorgeschlagen wurde. Jedoch sind die vorgeschlagenen Strukturen schwierig zugänglich und nur über die Acrylsäureestergruppe funktionalisierbar.

Der Erfindung liegt die Aufgabe zugrunde, Zusammensetzungen bereitzustellen, die eine mit Methacrylaten vergleichbare radikalische Polymerisationsreaktivität zeigen und bei der Polymerisation wenig schrumpfen.

Diese Aufgabe wird durch Zusammensetzungen gelöst, die ein Cyclopropylacrylat der allgemeinen Formel (1) 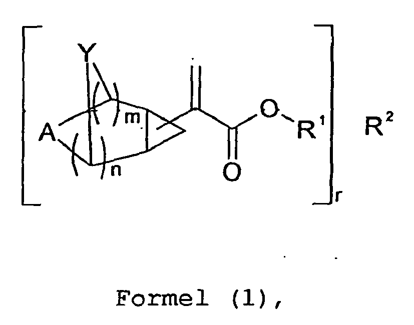 in der A ausgewählt ist aus 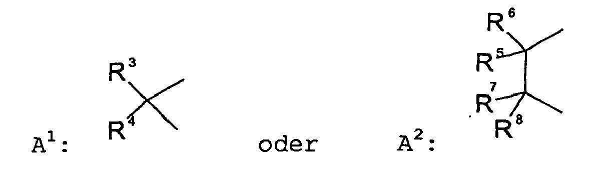 und
und die übrigen Variablen unabhängig voneinander die folgenden Bedeutungen haben:
- Y =: für A = A¹: entfällt, CH₂ oder O,
für A = A²: CH₂ oder O,
- n =: für Y entfällt: 0, 1, 2 oder 3,
für Y = CH₂ oder O: 1,
- m =: für Y entfällt: 0, 1, 2 oder 3,
für Y = CH₂ oder O: 1,
- r =: 1, 2, 3 oder 4,
- R¹ =: H, ein C₁- bis C₁₀-Alkyl-, C₆- bis C₁₂-Aryl-, C₁₋ bis C₁₀-Arylalkyl- oder bicyclischer C₅-C₁₂-Rest oder -R¹⁰-X-,
- R² =: ein durch den Klammerausdruck r-fach substitu- ierter aliphatischer C₁-C₂₀-Kohlenwasserstoff- rest, der durch O oder S unterbrochen sein kann, cycloaliphatischer C₄-C₁₂-Rest, bicyclischer C₅- C₁₂-Rest, C₆-C₁₄-Aryl- oder C₇-C₂₀-Alkylaryl-Rest,
- R³ =: -CO-OR⁹, -CO-R⁹, -S(O)R⁹, -SO₂R⁹, -SO₂(OR⁹), -PO(OR⁹)₂, -CN, -H, -R⁹,
- R⁴ =: -CO-OR⁹, -CO-R⁹, -S(O)R⁹, -SO₂R⁹, -SO₂(OR⁹), -PO(OR⁹)₂, -CN, oder -R¹¹-R¹⁰-X-
- R⁵-R⁸ =: unabhängig voneinander H, -CO-OR⁹, -CO-NHR⁹, -CO-NR⁹₂, -CO-R⁹, -CN, ein C₁-C₂₀-Alkyl-Rest, der durch O oder S unterbrochen sein kann, ein cy- cloaliphatischer C₄-C₁₂-Rest, ein bicyclischer C₅-C₁₂-Rest, ein C₆-C₁₄-Aryl-, C₇-C₂₀-Alkylaryl- Rest, oder mindestens zwei der Reste bilden zu- sammen mit den Kohlenstoffatomen, an die sie ge- bunden sind, ein 5- bis 8-gliedriges Ringsystem,
- R⁹ =: ein C₁-C₂₀-Alkyl-Rest, der durch O oder S unter- brochen sein kann, ein cycloaliphatischer C₄-C₁₂- Rest, in bicyclischer C₅-C₁₂-Rest, ein C₆-C₁₄- Aryl-, oder C₇-C₂₀-Alkylaryl-Rest,
- R¹⁰ =: entfällt oder ein C₁-C₂₀-Alkylen-Rest, der durch O oder S unterbrochen sein kann, ein cycloali- phatischer oder bicyclischer C₅-C₁₂-Rest, ein C₆- C₁₄-Arylen- oder C₇-C₂₀-Alkylenarylen-Rest,
- R¹¹ =: -COO-, -CO-, -SO-, -SO₂-, -SO₂(O-), -PO(OR⁹)(O-),
- X =: entfällt, -O-CO-, -CO-O-, -NH-CO-, -CO-NH-, -NH-CO-O- oder -O-CO-NH-, wobei X die Bedeutung "entfällt" hat, wenn R¹⁰ entfällt, und
wobei die Reste R² und R⁵⁻⁹ substituiert oder unsubstituiert sein können, entweder R⁴ = -R¹¹-R¹⁰-X- oder R¹ = -R¹⁰-
X- ist und der Klammerausdruck über X an R² gebunden ist, oder eine Mischung, davon, und einen Initiator für die radikalische Polymerisation enthalten.

Enthält das Cyclopropylacrylat der allgemeinen Formel (1) mehrere Reste eines Typs, beispielsweise mehrere R⁹-Reste, so können diese gleich oder verschieden sein.

Die Substituenten der Reste R² und R⁵⁻⁹ sind ggf. aus Alkyl, Halogen, OCH₃, OC₂H₅, Vinyl, (Meth) acryl, COOR¹², SiCl₃, Si(OR¹³)₃, und/oder mesogene Gruppen ausgewählt, wobei
- R¹² =: H oder C₁- bis C₁₀-Alkyl-, C₆- bis C₁₂-Aryl-, C₆- bis C₁₀-Arylalkyl- oder ein bicyclischer C₅-C₁₂- Rest und
- R¹³ =: H oder C₁- bis C₁₀-Alkyl-Rest.

Besonders bevorzugte Substituenten sind C₁-C₃-Alkyl, OCH₃, (Meth)acryl oder Si(OR¹³)₃ mit R¹³ = Methyl oder Ethyl.

Durch die Formel (1) werden sämtliche stereoisomere Formen sowie Gemische verschiedener konstitutionsund stereoisomerer Formen, wie z.B. Racemate, erfaßt. Wie Formel (1) zu entnehmen ist, kann der Rest -C(=CH₂)-C(O)-O-R¹ über das Brückenatom oder vorzugsweise ein Brückenkopfatom an den Cyclopropanring gebunden sein. Die Formel erfaßt nur solche Verbindungen, die mit der chemischen Valenzlehre vereinbar sind.

Unter Arylalkylgruppen werden Alkylreste verstanden, die durch eine oder mehrere Arylgruppen substituiert sind und unter Alkylarylgruppen dementsprechend Arylgruppen, die durch einen oder mehrere Alkylreste substituiert sind. Ein Alkylenarylenrest ist eine Gruppe, die sich aus mindestens einer Alkylengruppe und mindestens einer Arylengruppe zusammensetzt und sowohl am Alkylen- als auch am Arylenteil ungesättigt ist, wie beispielsweise -CH₂-Ph-.

Die Angabe, daß ein Rest durch Fremdatome, wie Sauerstoff oder Schwefel, unterbrochen sein kann, ist so zu verstehen, daß eines oder mehrere der Fremdatome in eine Kohlenstoffkette integriert sind. Daraus folgt, daß die Fremdatome nicht endständig sein können, d.h. eine Anbindung an Nachbargruppen immer über ein Kohlenstoffatom erfolgt, und daß die Zahl der Fremdatome zwangsläufig kleiner als die Zahl der Kohlenstoffatome sein muß.

Wenn Y entfällt, werden die Bindungsstellen der Ringatome durch Wasserstoff abgesättigt.

Bevorzugte Cyclopropylacrylate der Formel (1) sind Verbindungen bei denen A = A¹, R¹ = -R¹⁰-X- und Y entfällt (Formel 2), Verbindungen, bei denen A = A¹, R⁴ = -R¹¹-R¹⁰-X- und Y entfällt (Formel 3) und Verbindungen bei denen A = A², R¹ = -R¹⁰-X- und Y CH₂ oder O ist (Formel 4).

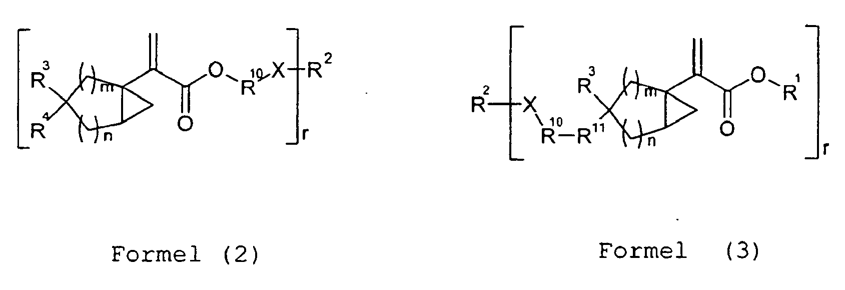

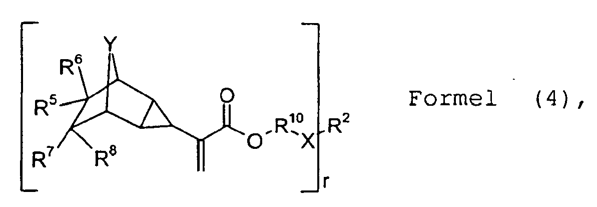

Die übrigen Variablen weisen die oben bei Formel (1) genannten Bedeutungen auf. Besonders bevorzugt sind Verbindungen der Formeln (2) und (3), ganz besonders bevorzugt Verbindungen der Formel (2).

Für die Variablen der Formel (1), (2), (3) und (4) existieren die folgenden unabhängig voneinander wählbaren bevorzugten Definitionen:
- Y =: für A = A¹: entfällt, für A = A²: CH₂ oder O,
- n =: 1,
- m =: 1,
- r =: 1 oder 2,
- R¹ =: H, C₁- bis C₅-Alkyl- oder bicyclischer C₅-C₁₂- Rest oder insbesondere -R¹⁰-X-,
- R² =: ein aliphatischer C₁-C₆-Kohlenwasserstoffrest, der durch O unterbrochen sein kann, ein cycloa- liphatischer C₆-C₈-Rest, ein bicyclischer C₆-C₈- Rest, ein C₆-C₁₀-Aryl- oder C₇-C₁₀-Alkylaryl-Rest,
- R³ =: -CO-OR⁹, CO-R⁹, -SO₂R⁹, CN, H oder -R⁹,
- R⁴ =: -CO-OR⁹, -CO-R⁹, -SO₂R⁹, CN oder -R¹¹-R¹⁰-X-
- R⁵-R⁸ =: unabhängig voneinander H, -CO-OR⁹, -CO-R⁹, CN, ein C₁-C₆-Alkyl-Rest, der durch O unterbrochen sein kann, ein cycloaliphatischer C₆-C₈-Rest, ein bicyclischer C₆-C₈-Rest, ein C₆-C₁₀-Aryl- oder C₇-C₁₀-Alkylaryl-Rest,
- R⁹ =: ein C₁-C₆-Alkyl-Rest, der durch O unterbrochen sein kann, ein cycloaliphatischer C₆-C₈-Rest, ein bicyclischer C₆-C₈-Rest, ein C₆-C₁₀-Aryl- oder C₇-C₁₀-Alkylaryl-Rest, insbesondere C₁-C₃- Alkyl,
- R¹⁰ =: entfällt oder ein C₁-C₁₀-Alkylen-Rest, der durch O unterbrochen sein kann, ein bicyclischer C₆- C₉-Rest, oder C₇-C₁₀-Alkylenarylen-Rest, insbe- sondere entfällt oder C₁-C₆-Alkylen,
- R¹¹ =: -CO-O-, -CO- oder -SO₂-, insbesondere -CO-O-,
- X =: entfällt, -O-CO- oder -CO-O-.

Besonders bevorzugt sind solche Cyclopropylacrylate, bei denen alle Variablen eine der bevorzugten Bedeutungen aufweisen.

Die erfindungsgemäßen Cyclopropylacrylate der allgemeinen Formel (2) (R¹⁰, X entfällt, m=1, n=1, r=1) können ausgehend von O-Alkyl-O'-(4-chlor-but-2-inyl)-carbonaten erhalten werden:

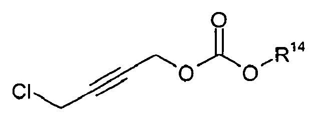

Diese können einfach durch Überführung von But-2-in-1,4-diol in das Monochlorid mittels Thionylchlorid und nachfolgende Umsetzung mit Chlorameisensäureestern hergestellt werden (A.G. Steinig, Dissertation, Universität Göttingen, 1997). Durch Umsetzung mit geeigneten 4,4-disubstituierten But-1-enen erhält man 5,5-disubstituierte O-Alkyl-O'-(oct-7-en-2-inyl)-carbonate (A. G. Steinig, Dissertation, Universität Göttingen, 1997).

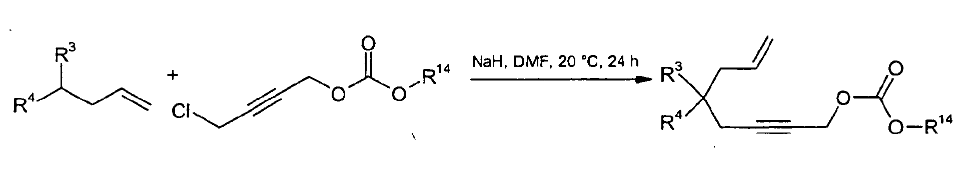

Alternativ lassen sich die erfindungsgemässen Cyclopropylacrylate der allgemeinen Formel (2) (R¹⁰, X entfällt, m=1, n=1, r=1) in drei Stufen ausgehend von käuflichem 4-Chlor-2-butin-1-ol hergestellen. Dieses ergibt durch Umsetzung mit geeigneten 4,4-disubstituierten But-1-enen und nachfolgende Veresterung mit Chlorameisensäuremethylester entsprechende 5,5-disubstituierte O-Alkyl-O'-(oct-7-en-2-inyl)-carbonate.

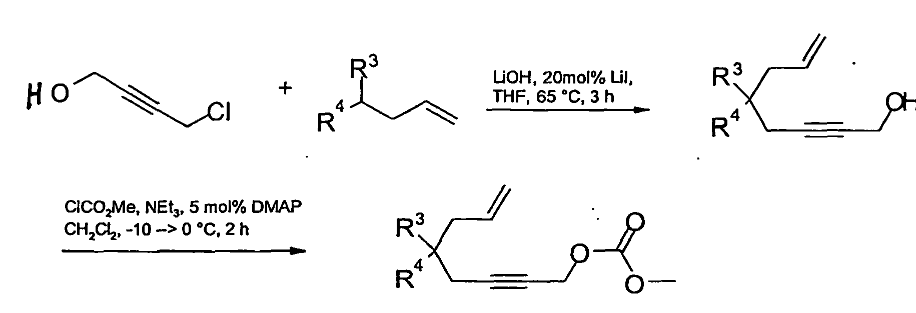

Der nächste Schritt ist eine palladium-katalysierte vierstufige Kaskadenreaktion, bei der zunächst der Fünf-, dann der Dreiring gebildet wird, und letztlich der gebildete Vinylpalladium-Komplex mit Kohlenmonoxid und dann einem Alkohol zu dem 3,3-disubstituierten 2-(Bicyclo[3.1.0]hex-1-yl)-acrylsäureester der allgemeinen Formel (2) reagiert (R. Grigg, R. Rasul, J. Redpath, D. Wilson, Tetrahedron Lett. 37 (1996) 4609; W. Oppolzer, A. Pimm, B. Stammen, E. Hume, Helv. Chim. Acta, 80 (1997) 623:

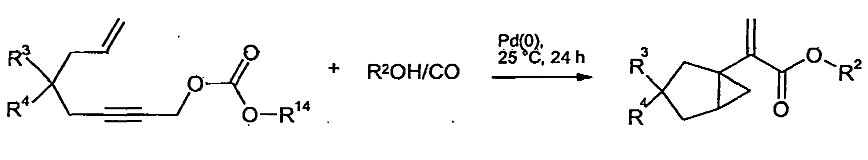

### Konkretes Beispiel:

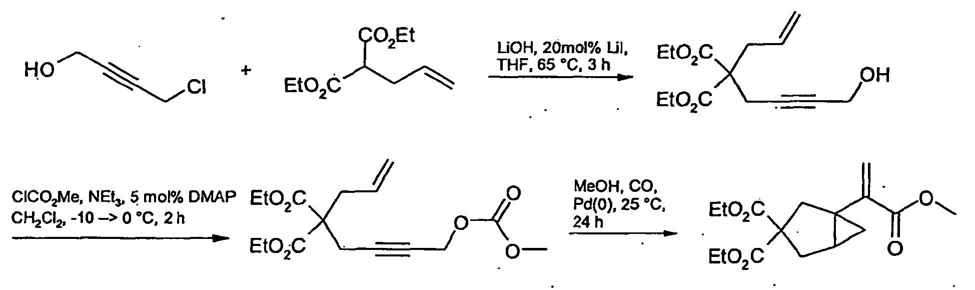

Cyclopropylacrylate der allgemeinen Formel (2) (r >1) können durch Hydrolyse von Cyclopropylacrylsäuremethylestern (r=1 und R¹⁰, X = entfällt) und nachfolgende Veresterung mit polyfunktionellen Alkoholen [(HO-R¹⁰-X-)ᵣ] erhalten werden:

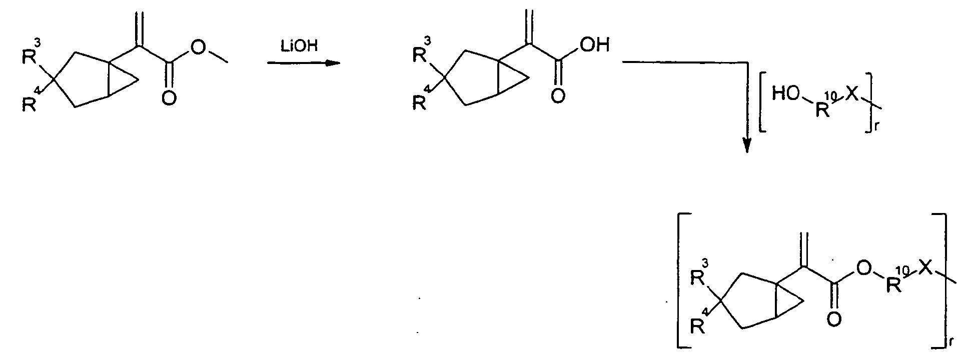

### Konkretes Beispiel:

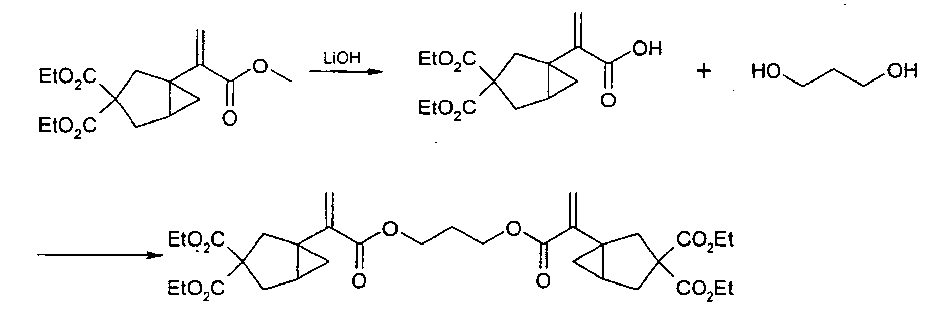

Cyclopropylacrylate der allgemeinen Formel (3) (r >1, R¹¹ = CO-O) können durch Hydrolyse von Cyclopropylacrylaten (r=1 und R¹⁰, X = entfällt, R¹¹ = CO-O) und nachfolgende Veresterung mit oligofunktionellen Alkoholen [(HO-R¹⁰-X-)ᵣ] erhalten werden:

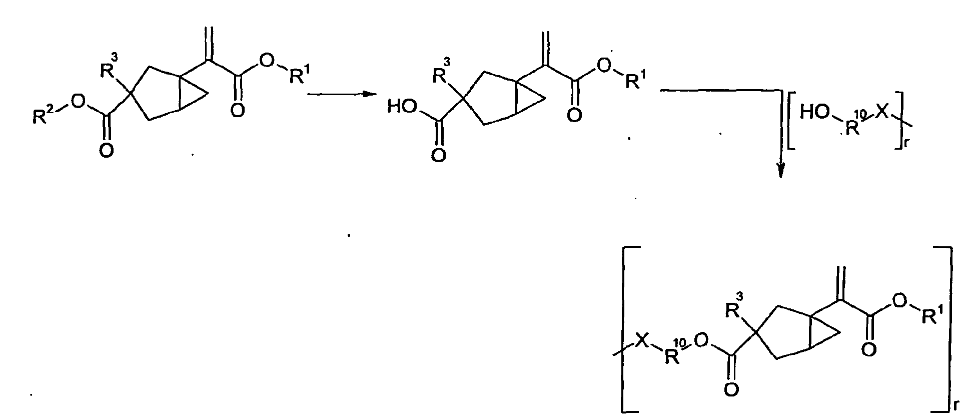

### Konkretes Beispiel:

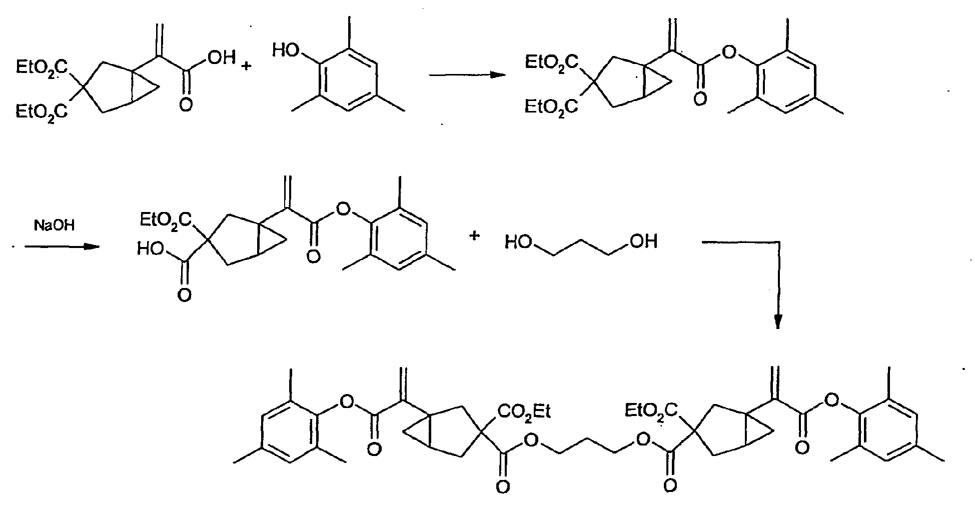

Cyclopropylacrylate der allgemeinen Formel (4) (r = 1, R¹⁰, X entfällt) können durch Cyclopropanierung von 5,6-substituierten Bicyclo[2.2.1]hept-2-enen mit Diazopyruvaten und nachfolgende WITTIG-Olefinierung erhalten werden (L. G. Mueller, R. G. Lawton, J. Org. Chem. 44 (1979) 4714; E. Wenkert, Helv. Chim. Acta 70 (1987) 2159:

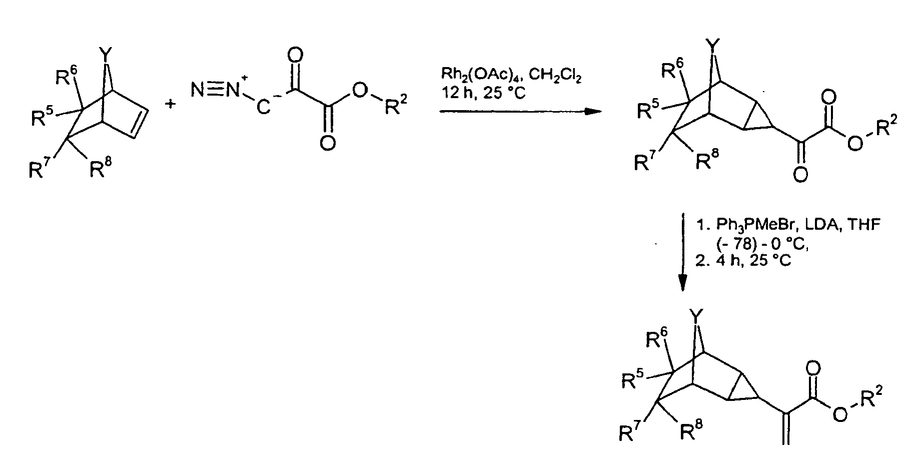

### Konkretes Beispiel:

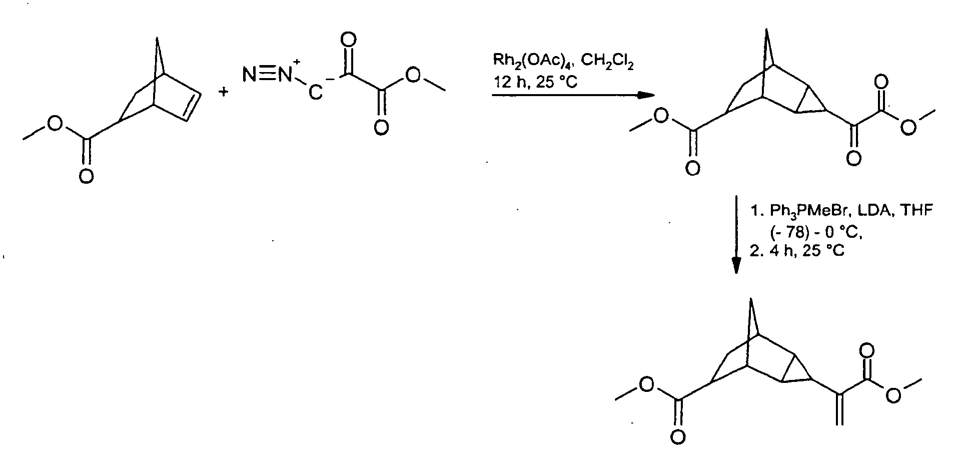

Bevorzugte Beispiele für die erfindungsgemäßen Cyclopropylacrylate der Formel (1) sind:

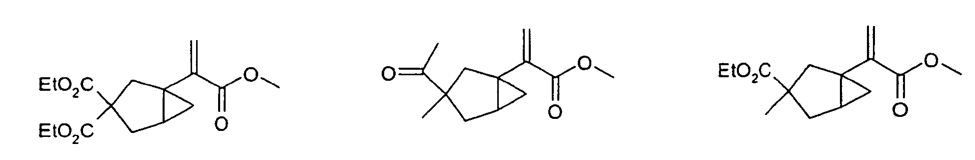

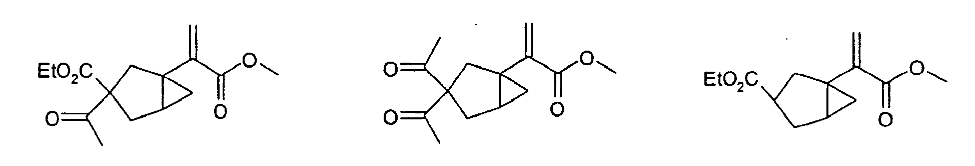

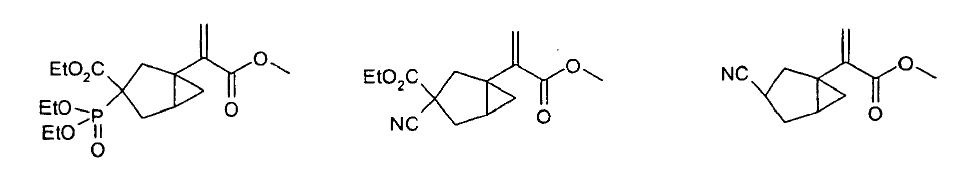

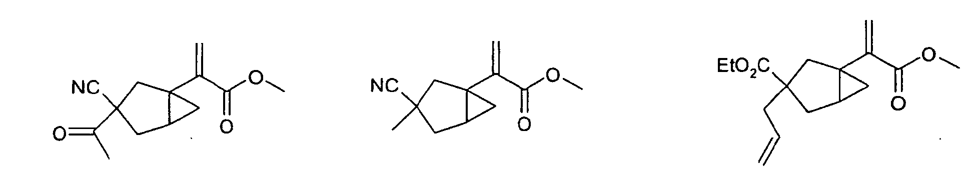

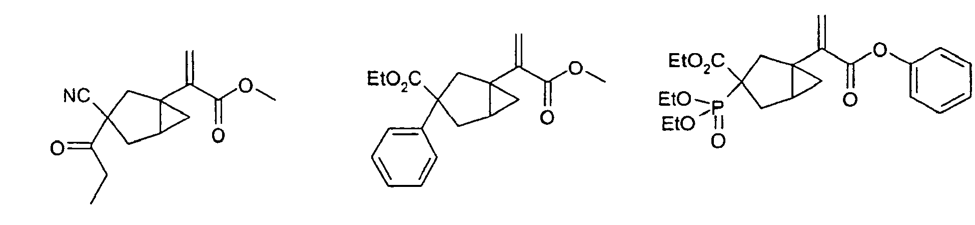

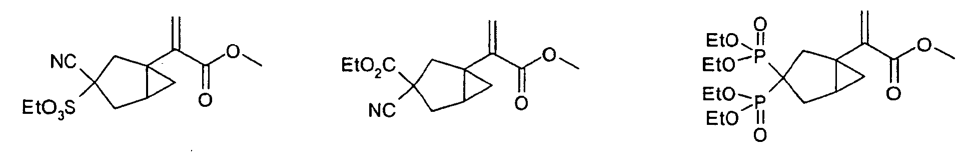

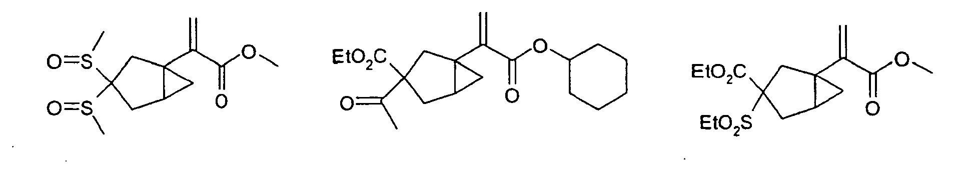

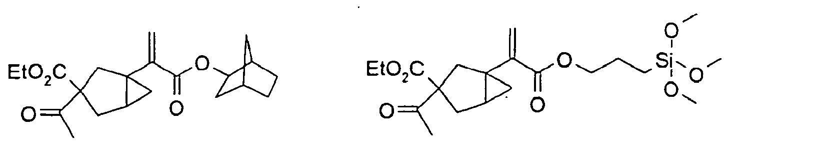

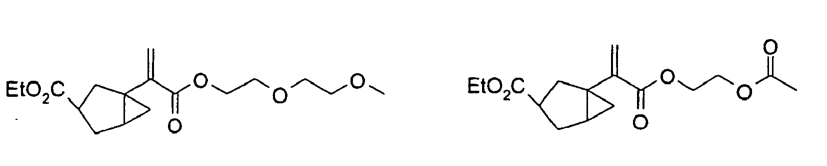

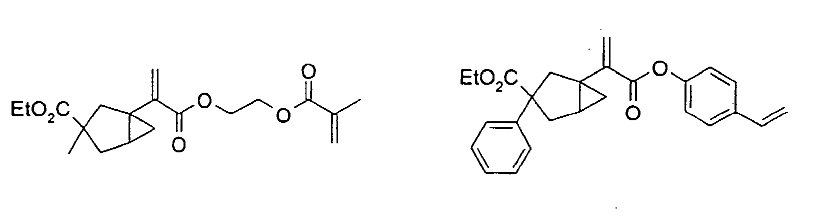

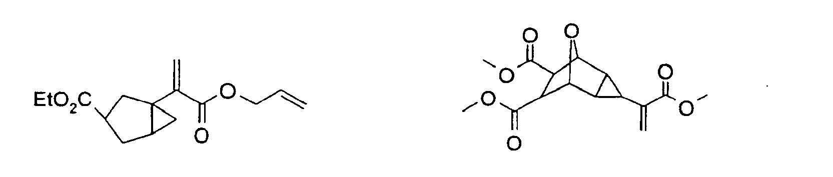

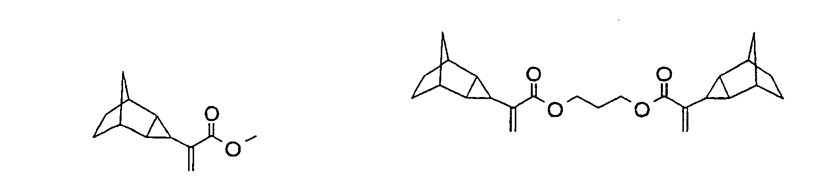

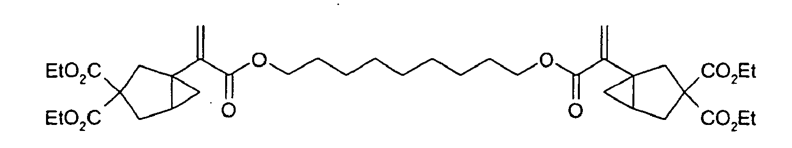

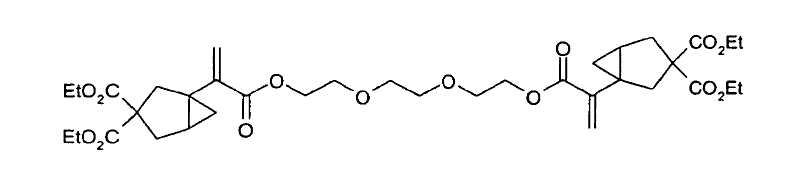

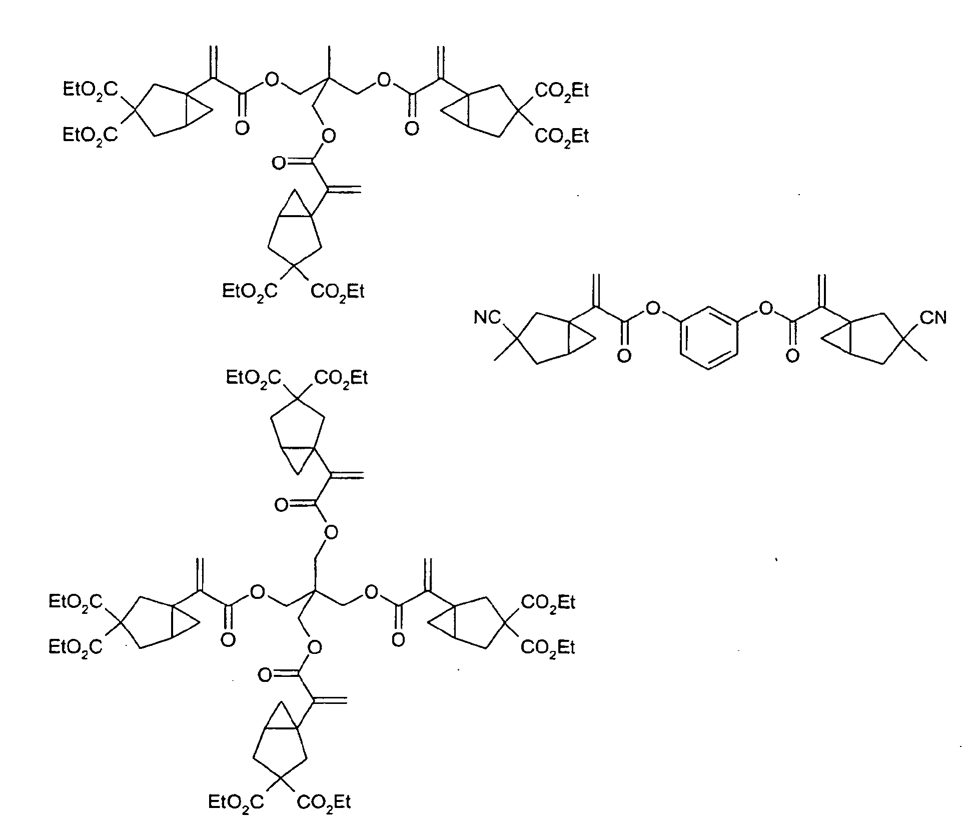

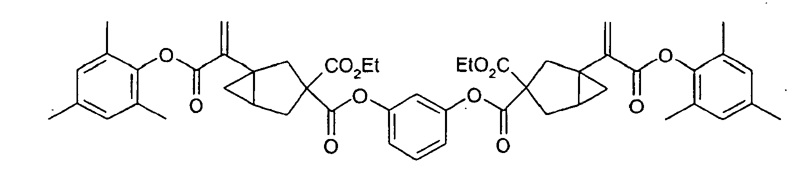

Die vorliegende Erfindung betrifft Zusammensetzungen, die Cyclopropylacrylate gemäß Formel (1) enthalten.

Die Cyclopropylacrylate der Formel (1) eignen sich besonders zur Herstellung von Dentalmaterialien, Polymeren und Copolymeren, Formkörpern, Adhäsiven, Zementen, Füllmaterialien, Beschichtungsmaterialien und Kompositen insbesondere zur dentalen Anwendung.

Hierzu werden sie mit einem Initiator für die radikalische Polymerisation und vorzugsweise auch mit zusätzlichen Monomeren Füllstoffen und ggf. weiteren Hilfsstoffen gemischt. Die so erhaltenen Zusammensetzungen können durch radikalische Polymerisation gehärtet werden. Sowohl die gehärteten Produkte, wie z.B. Polymere und Formkörper, als auch die härtbaren Zusammensetzungen sind Gegenstand der Erfindung.

Die Zusammensetzungen der Erfindung enthalten zusätzlich zu dem Cyclopropylacrylat einen Initiator für die radikalische Polymerisation. Die Cyclopropylacrylate der Formel (1) lassen sich mit bekannten radikalischen Initiatoren (vgl. Encylopedia of Polymer Science and Engineering, Vol. 13, Wiley-Intersci. Pub., New York etc. 1988, 754ff.) unter Ringöffnung polymerisieren. Als Initiatoren für die radikalische Polymerisation eignen sich bevorzugt Azoverbindungen, wie 2,2'-Azobis(isobutyronitril) (AIBN) oder Azobis-(4-cyanovaleriansäure) oder Peroxide, wie Dibenzoylperoxid, Dilauroylperoxid, tert.-Butylperoctoat, tert.-Butylperbenzoat oder Di-(tert.-butyl)-peroxid.

Als Initiatoren für die Heißhärtung eignen sich besonders Benzpinakol und 2,2'-Dialkylbenzpinakole.

Weiterhin können auch Photoinitiatoren (vgl. J.P. Fouassier, J.F. Rabek (Hrsg.), Radiation Curing in Polymer Science and Technology, Vol. II, Elsevier Applied Science, London und New York 1993) für den UV- oder sichtbaren Bereich, wie Benzoinether, Dialkylbenzilketale, Dialkoxyacetophenone, Acyloder Bisacylphosphinoxide, α-Diketone wie 9,10-Phenanthrenchinon, Diacetyl, Furil, Anisil, 4,4'-Dichlorbenzil, 4,4'-Dialkoxybenzil und Campherchinon verwendet werden. Bevorzugt werden Campherchinon und 2,2-Dimethoxy-2-phenyl-acetophenon und besonders bevorzugt α-Diketone, wie Campherchinon in Kombination mit Aminen als Reduktionsmittel, wie z.B. 4-(N,N-Dimethylamino)-benzoesäureester, N,N-Dimethylaminoethylmethacrylat, N,N-Dimethyl-sym.-xylidin oder Triethanolamin, eingesetzt. Darüber hinaus sind auch Acylphosphine, wie z.B. 2,4,6-Trimethylbenzoyldiphenyl- oder Bis(2,6-dichlorbenzoyl)-4-n-propylphenylphosphinoxid besonders geeignet.

Als Initiatoren für eine bei Raumtemperatur durchgeführte Polymerisation werden Redox-Initiatorkombinationen, wie z.B. Kombinationen von Benzoyl- oder Lauroylperoxid mit N,N-Dimethyl-sym.-xylidin oder N,N-Dimethyl-p-toluidin, verwendet. Darüber hinaus sind auch Redoxsysteme, bestehend aus Peroxiden und Reduktionsmitteln, wie z.B. Ascorbinsäure, Barbiturate oder Sulfinsäuren, geeignet.

Die erfindungsgemäßen Cyclopropylacrylate lassen sich allein oder in Mischung mit herkömmlichen radikalisch polymerisierbaren Monomeren, insbesondere mit difunktionellen oder mehrfunktionellen Vernetzermonomeren polymerisieren. Vernetzermonomere sind Verbindungen mit zwei oder mehr radikalisch polymerisierbaren Gruppen. Bevorzugt sind Monomere mit zwei bis drei polymerisierbaren Gruppen.

Somit können erfindungsgemäße Zusammensetzungen zusätzlich mindestens ein weiteres radikalisch polymerisierbares Monomer, vorzugsweise ein mehrfunktionelles Monomer enthalten.

Für die Herstellung von Adhäsiven, Beschichtungsmaterialien oder Dentalmaterialien eignen sich bevorzugt Zusammensetzungen, die Mischungen eines oder mehrerer der Cyclopropylacrylate mit mindestens einem bi- oder mehrfunktionellen Acrylat oder Methacrylat enthalten, wie z.B. Bisphenol-A-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat und 2,2,4-Trimethylhexamethylendiisocyanat), Di-, Tri- oder Tetraethylenglycoldi(meth)acrylat, Trimethylolpropantri(meth)acrylat, Pentaerythrittetra(meth)acrylat, sowie Butandioldi(meth)acrylat, 1,10-Decandioldi(meth)acrylat und 1,12-Dodecandioldi(meth)acrylat.

Weitere mehrfunktionelle radikalisch polymerisierbare Monomere, die in erfindungsgemäßen Zusammensetzungen enthalten sein können, sind z.B. Urethane aus 2-(Hydroxymethyl)acrylsäure und Diisocyanten, wie 2,2,4-Trimethylhexamethylendiisocyanat oder Isophorondiisocyanat, vernetzende Pyrrolidone, wie z.B. 1,6-Bis(3-vinyl-2-pyrrolidonyl)-hexan, oder kommerziell zugängliche Bisacrylamide wie Methylen- oder Ethylenbisacrylamid, bzw. Bis(meth)acrylamide, wie z.B. N,N'-Diethyl-1,3-bis(acrylamido)-propan, 1,3-Bis(methacrylamido)-propan, 1,4-Bis(acrylamido)-butan oder N,N'-Bis-(acryloyl)-piperazin, die durch Umsetzung aus den entsprechenden Diaminen mit (Meth)acrylsäurechlorid synthetisiert werden können.

Weiterhin können die erfindungsgemäß eingesetzten Zusammensetzungen zur Verbesserung der mechanischen Eigenschaften oder zur Einstellung der Viskosität Füllstoffe, vorzugsweise organische oder anorganische Partikel enthalten. Bevorzugte anorganische partikuläre Füllstoffe sind amorphe kugelförmige Materialien auf der Basis von Oxiden, wie ZrO₂ und TiO₂ bzw. Mischoxiden aus SiO₂, ZrO₂ und/oder TiO₂, nanopartikuläre Füllstoffe sowie Minifüllstoffe. Unter nanopartikulären Füllstoffen versteht man Füllstoffe mit einer Primärpartikelgrösse von ca. 5 bis 100 nm, wie z.B. Aerosil 200 mit einer Primärpartikelgrösse von 12 nm. Ebenso werden als anorganische partikuläre Füllstoffe bevorzugt Minifüllstoffe, d.h.Füllstoffe mit einer Partikelgrösse zwischen 0,1 und 1,5 µm, wie z.B. fein gemahlene Quarz-, Glaskeramik- oder Glaspulver, sowie röntgenopake Füllstoffe, wie Ytterbiumtrifluorid, nanopartikuläres Tantal(V)-oxid oder Bariumsulfat, verwendet. Darüber hinaus können auch Glasfasern, Polyamid- oder Kohlenstoff-Fasern eingesetzt werden.

Außerdem können die erfindungsgemäßen Zusammensetzungen im Bedarfsfalle weitere Komponenten enthalten, wie z.B. Stabilisatoren, UV-Absorber, Farbstoffe oder Pigmente sowie Gleitmittel.

Die erfindungsgemäßen Zusammensetzungen eignen sich besonders als Dentalmaterialien, insbesondere als Dentaladhäsive, Befestigungszemente oder Füllungsmaterialien sowie Materialien für Inlays/Onlays, Zähne oder Verblendmaterial für Kronen und Brücken. Solche Materialien zeichnen sich durch einen geringeren Polymerisationsschrumpf und sehr gute mechanische Eigenschaften nach der Härtung aus.

Zur Verwendung als Adhäsiv eignen sich besonders Zusammensetzungen, die
a) 1 bis 80 Gew.-%, und besonders bevorzugt 10 bis 60 Gew.-% erfindungsgemäßes Cyclopropylacrylat,
b) 0,01 bis 5 Gew.-%, besonders bevorzugt 0,1 bis 2,0 Gew.-% Initiator für die radikalische Polymerisation,
c) 0 bis 60 Gew.-% und besonders bevorzugt 0 bis 40 Gew.-% weiteres radikalisch polymerisierbares Monomer,
d) 0 bis 40 Gew.-% und besonders bevorzugt 0 bis 30 Gew.-% Lösungsmittel und
e) 0 bis 20 Gew.-% Füllstoff
enthalten.

Zur Verwendung als Zement eignen sich besonders Zusammensetzungen, die
a) 1 bis 60 Gew.-%, und besonders bevorzugt 20 bis 50 Gew.-% erfindungsgemäßes Cyclopropylacrylat,
b) 0,01 bis 5 Gew.-%, besonders bevorzugt 0,1 bis 2,0 Gew.-% Initiator für die radikalische Polymerisation,
c) 0 bis 60 Gew.-% und besonders bevorzugt 0 bis 20 Gew.-% weiteres radikalisch polymerisierbares Monomer und
d) 20 bis 60 Gew.-% und besonders bevorzugt 30 bis 60 Gew.-% Füllstoff
enthalten.

Zur Verwendung als Füllungsmaterial eignen sich besonders Zusammensetzungen, die
a) 1 bis 45 Gew.-%, und besonders bevorzugt 10 bis 30 Gew.-% erfindungsgemäßes Cyclopropylacrylat,
b) 0,01 bis 5 Gew.-%, besonders bevorzugt 0,1 bis 2,0 Gew.-% Initiator für die radikalische Polymerisation,
c) 0 bis 50 Gew.-% und besonders bevorzugt 0 bis 10 Gew.-% weiteres radikalisch polymerisierbares Monomer und
d) 30 bis 85 Gew.-% und besonders bevorzugt 40 bis 80 Gew.-% Füllstoff
enthalten.

Zur Verwendung als Beschichtungsmaterial eignen sich besonders Zusammensetzungen, die
a) 1 bis 95 Gew.-%, und besonders bevorzugt 10 bis 60 Gew.-% erfindungsgemäßes Cyclopropylacrylat,
b) 0,01 bis 5 Gew.-%, besonders bevorzugt 0,1 bis 2,0 Gew.-% Initiator für die radikalische Polymerisation,
c) 0 bis 60 Gew.-% und besonders bevorzugt 0 bis 40 Gew.-% weiteres radikalisch polymerisierbares Monomer und
d) 0 bis 20 Gew.-% Füllstoff
enthalten.

Im folgenden wird die Erfindung anhand von Ausführungsbeispielen näher erläutert.

### Ausführungsbeispiele

### Beispiel 1: {3,3-Bis(ethoxycarbonyl)bicyclo[3.1.0]hex-1-yl}acrylsäuremethylester

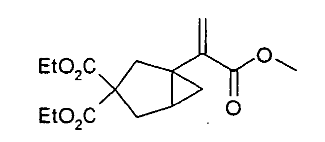

### 1. Stufe: 2-Allyl-2-(4-hydroxybut-2-inyl)malonsäurediethylester

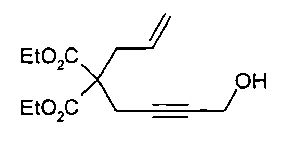

Zu einer gerührten Suspension von LiOH (2,51 g, 105 mmol) und LiI (2,68 g, 20 mmol) in wasserfreiem Tetrahydrofuran (THF, 50 ml) wurde unter Stickstoff 2-Allyl-malonsäureethylester (20 g, 100 mmol) in einer Portion zugesetzt und das Reaktionsgemisch unter Rückfluss erhitzt bis es homogen war (etwa 30 Minuten). Nach Abkühlen auf Raumtemperatur wurde 4-Chlor-2-butin-1-ol (11,5 g, 110 mmol) zugesetzt und die Mischung weitere 3 h unter Rühren und Rückfluss erhitzt. Man ließ erneut auf Raumtemperatur abkühlen, versetzte mit 25 ml Wasser und extrahierte dreimal mit je 50 ml Essigsäureethylester. Die vereinigten Extrakte wurden mit 50 ml gesättigter Kochsalzlösung gewaschen und über Magnesiumsulfat getrocknet. Nach Abdampfen der Lösungsmittel im Vakuum erhielt man 27 g Rohprodukt, das bei 0,001 mbar fraktioniert destilliert wurde. Die zwischen 140 und 145 °C siedende Fraktion war reines Produkt. Ausbeute: 17,5 g (63%, leicht gelbliches viskoses Öl).

IR (Film): ν = 3471, 3083, 2985, 2936, 2909, 2876, 2227, 1731, 1644, 1464, 1442, 1387, 1365, 1322, 1289, 1251, 1213, 1191, 1131, 1093, 1066, 1032, 1016, 924, 858, 782, 653, 580 cm⁻¹.

¹H-NMR (250 MHz, CDCl₃) : δ = 1.24 (t, ³*J* = 7.3 Hz, 6 H, 2 CH₃), 1.90 (s, 1 H, OH), 2.77 (ddd, ³*J* = 7.5, ⁵*J* = 0.9, 0.9 Hz, 2 H, =CHC*H₂*), 2.81 (t, ⁵*J* = 2.2 Hz, 2 H, =CCH₂), 4.19 (q, ³*J* = 7.3 Hz, 4 H, 2 OCH₂), 4.21 (t, ⁵*J* = 2.2 Hz, 2 H, =CCH₂O), 5.11 (ddt, ³*J* = 10, ²*J* = 1.4, ⁵*J* = 0.9 Hz, 1 H, =CH₂), 5.17 (ddt, ³*J* = 17, ²*J* = 1.4, ⁵*J* = 0.9 Hz, 1 H, =CH₂), 5.61 (ddt, ³*J* = 17, 10, 7.5 Hz, 1 H, -CH=) ppm.

¹³C-NMR (62. 9 MHz, CDCl₃, DEPT): δ = 14.0 (2 CH₃), 22.8 (CH₂), 36.4 (CH₂), 51.1 (CH₂), 56.7 (C), 61.3 (2 CH₂), 80.5 (C), 81.5 (C), 119.8 (CH₂), 131.7 (CH), 169.8 (2 C) ppm.

MS (70 eV, EI) : *m*/*z* (%) = 268 (1) [M⁺], 251 (6) [M⁺ - OH], 239 (9) [M⁺ - C₂H₅], 227 (21) [M⁺ - C₃H₅], 223 (6) [M⁺ - C₂H₅O], 219 (6), 205 (18) [M⁺ - C₂H₅O - OH - 2 H], 199 (43) [M⁺ - OH - C₄H₃ - H], 195 (32) [M⁺ - C₂H₅O - CO], 181 (34) [M⁺ - C₂H₅O - C₃H₅ - H], 177 (92) [M⁺ - C₂H₅O - CO - OH - H], 173 (25), 165 (22), 157 (13), 153 (100) [M⁺ - C₂H₅O - CO - C₃H₅ - H], 149 (74) [M⁺ - 2 C₂H₅ - CO - H], 137 (11), 135 (38), 133 (25) [M⁺ - 2 C₂H₅O - CO - OH], 127 (11), 125 (41), 121 (68) [M⁺ - 2 C₂H₅O - 2 CO - H], 105 (60) [C₈H₉⁺], 103 (90) [C₈H₇⁺], 93 (42) [C₇H₉⁺], 91 (66) [C₇H₇⁺], 79 (30), 77 (42), 71 (6), 67 (7), 65 (13), 55 (11), 53 (12), 51 (5), 43 (6), 41 (18).

C₁₂H₁₆O₃ (208.25): ber. C 62.67, H 7.51; gef. C 62.42, H 7.28.

### 2. Stufe: Kohlensäure-O-[5,5-bis(ethoxycarbonyl)oct-7-en-2-in-yl]-O'-methylester

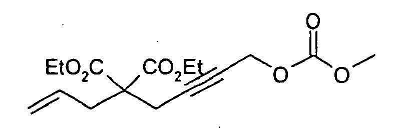

Zu einer gerührten Lösung von 2-Allyl-2-(4-hydroxybut-2-inyl)malonsäurediethylester (18,9 g, 70 mmol), NEt₃ (10,3 ml, 74 mmol) und DMAP (0,43 g, 3,5 mmol) in 70 ml wasserfreiem Dichlormethan wurde bei -10 °C unter Stickstoff Chlorameisensäuremethylester (6.2 ml, 80 mmol, gelöst in 10 ml Dichlormethan) innerhalb einer Stunde getropft, wobei die Temperatur des Reaktionsgemisches unter -5 °C gehalten wurde. Dann wurde noch für weitere 30 Minuten bei dieser Temperatur gerührt, das Kühlbad entfernt und der größte Teil des Lösungsmittels im Vakuum bei maximal Raumtemperatur entfernt. Der Rückstand wurde mit 35 ml Wasser und 70 ml Diethylether versetzt, die organische Phase abgetrennt und die wässrige Phase noch mit 2 × 30 ml Diethylether extrahiert. Die vereinigten organischen Phasen wurden über Magnesiumsulfat getrocknet. Nach dem Abziehen des Lösungsmittels erhielt man 23,5 g Rohprodukt, das durch Flash-Chromatographie gereinigt wurde (250 ml Flash-Kieselgel, Elutionsmittel: Pentan/Diethylether: 10:1 bis 3:1. Ausbeute: 19,8 g (87%, farbloses Öl).

IR (Film) : ν = 3081, 2983, 2963, 2941, 2908, 2875, 2239, 2156, 2046, 1755, 1734, 1640, 1443, 1370, 1328, 1267, 1218, 1189, 1139, 1096, 1069, 1031, 954, 901, 861, 792 cm⁻¹.

¹H-NMR (250 MHz, CDCl₃): δ = 1,23 (t, ³*J* = 7,2 Hz, 6 H, 2 CH₃), 2,76 (ddd, ³*J* = 7,4 Hz, ⁴*J* = 1,5 Hz, ⁴*J* = 1 Hz, 2 H, 6-H), 2,82 (t, ⁵*J* = 2,1 Hz, 2 H, 4-H), 3,79 (s, 3 H, CH₃), 4,19 (q, ³*J* = 7,2 Hz, 4 H, 2 CH₂), 4,67 (t, ⁵*J* = 2,1 Hz, 2 H, 1-H), 5,10 (ddd, ³*J* = 9,9 Hz, ²*J* = 1,5 Hz, ⁴*J* = 1 Hz, 1 H, 8-H*ₜᵣₐₙₛ*),5, 15 (ddd, ³*J* = 17,3 Hz, ²*J* = 1,5 Hz, ⁴*J* = 1, 5 Hz, 1 H, 8-H*_{cis}*), 5, 60 (ddd, ³*J* = 17,3 Hz, ³*J* = 9,9 Hz, ³*J* = 7,4 Hz, 1 H, 7-H).

¹³C-NMR (62,9 MHz, CDCl₃, DEPT): δ = 14 , 0 (2 CH₃), 22,8 (CH₂), 36,4 (CH₂), 55,0 (CH₃), 55,8 (CH₂), 56,6 (C), 61,6 (2 CH₂), 76,5 (C), 82,8 (C), 119,8 (CH₂), 131,7 (CH), 155,1 (C), 169,6 (2 C).

MS (70 eV, DCI, NH₃), *m*/*z* (%): 670,5 (1) [2M + NH₄⁺], 344,3 (100) [M + NH₄⁺], 327,3 (9) [M + H⁺], 251,2 (4), 177 (2).

C₁₆H₂₂O₇ (326,34): ber. C 58,89, H 6,79; gef. C 58,58, H 7,08.

### 3. Stufe: {3,3-Bis(ethoxycarbonyl)bicyclo[3.1.0]hex-1-yl}acrylsäuremethylester

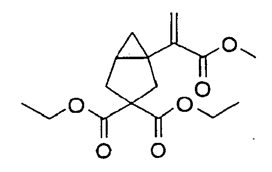

Zu einer Lösung von Tris(2-furyl)phosphan (505 mg, 2,2 mmol) und Tetramethylammoniumbromid (167 mg, 1,1 mmol) in entgasten Methanol (300 ml) gab man Pd(OAc)₂ (224 mg, 1,0 mmol) und rührte das Gemisch bei Raumtemperatur 1 Stunde lang. Dann setzte man Kohlensäure-O-[5,5-bis(ethoxycarbonyl)oct-7-en-2-inyl]-O'-methylester (7,35 g, 22,5 ml) hinzu und leitete Kohlenmonoxid ein. Die Mischung wurde 2 Tage bei Raumtemperatur unter 1 bar Kohlenmonoxid-Druck gerührt und dann das Lösungsmittel im Vakuum entfernt. Das Rohprodukt wurde chromatographisch an 100 ml Flash-Kieselgel mit Pentan/Äther (10:1 zu 5:1) gereinigt. Ausbeute: 6,03 g (86%), farbloses Öl.

IR (Film): ν = 3074, 2983, 2957, 2908, 2878, 1728, 1629, 1438, 1384, 1367, 1325, 1295, 1245, 1212, 1198, 1177, 1124, 1092, 1069, 1058, 1018, 1000, 955, 898, 861, 817, 766, 696 cm⁻¹.

¹H-NMR (250 MHz, CDCl₃): δ = 0,52 (dd, ²*J* = 5,6 Hz, ³*J* = 4,5 Hz, 1 H, 6-H*_{endo}*), 0, 74 (ddd, ³*J* = 7,9 Hz, ²*J* = 5,6 Hz, ⁴*J* = 1,5 Hz,

1 H, 6-H*ₑₓₒ*), 1,51-1,59 (m, 1 H, 1-H), 1,21 (t, ³*J* = 7,2 Hz, 3 H, CH₃), 1,24 (t, ³*J* = 7,2 Hz, 3 H, CH₃), 2,51-2,62 (m, 3 H), 2,71 (d, ²*J* = 13,5 Hz, 1 H), 3,75 (s, 3 H, OCH₃), 4,15 (q, ³*J* = 7,2 Hz, 2 H, OCH₂), 4,17 (q, ³*J* = 7,2 Hz, 2 H, OCH₂), 5,59 (d, ²*J* = 1,2 Hz, 1 H, 3'-H*ₜᵣₐₙₛ*), 6,15 (d, ²*J* = 1,2 Hz, 1 H, 3'-H_{cis}).

¹³C-NMR (62,9 MHz, CDCl₃, DEPT): δ = 13,9 (2 CH₃), 16,3 (CH₂), 24, 9 (CH), 31, 2 (C), 36, 0 (CH₂), 40, 2 (CH₂), 51,8 (CH₃), 59,9 (C), 61,6 (CH₂), 61,7 (CH₂), 125,8 (CH₂), 142,2 (C), 167,0 (C), 171,6 (C), 172,9 (C).

MS (70 eV, EI), *m*/*z* (%): 310 (8) [M⁺], 279 (17), 278 (55), 265 (36), 250 (25), 236 (20), 218 (4), 205 (15), 190 (55), 177 (100), 163 (32), 149 (18), 147 (26), 131 (43), 105 (26), 103 (81), 91 (12), 79 (12), 77 (20), 65 (3), 59 (4), 55 (4), 53 (3), 51 (2), 41 (3).

C₁₆H₂₂O₆ (310, 34) : ber. C 61, 92, H 7, 15; gef. C 61, 57, H 7, 36.

### Beispiel 2: 2-(3-Acetyl-3-ethoxycarbonyl-bicyclo[3.1.0]hex-1-yl)acrylsäuremethylester (ABHCE)

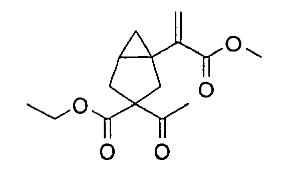

### 1. Stufe: 2-Acetyl-2-allyl-6-hydroxy-hex-4-insäureethylester

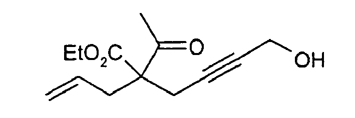

Zu einer gerührten Suspension von LiOH (5,03 g, 210 mmol) und LiI (5,35 g, 40 mmol) in wasserfreiem THF (100 ml) wurde unter Stickstoff 2-Allyl-acetessigsäureethylester (34,2 g, 200 mmol) in einer Portion zugesetzt und das Reaktionsgemisch unter Rückfluss erhitzt bis es homogen war (etwa 30 Minuten). Nach Abkühlen auf Raumtemperatur wurde 4-Chlor-2-butin-1-ol (23 g, 220 mmol) zugesetzt und die Mischung weitere 3 h unter Rühren am Rückfluss erhitzt. Man ließ erneut auf Raumtemperatur abkühlen versetzte mit 50 ml Wasser und extrahierte dreimal mit je 100 ml Essigsäureethylester. Die vereinigten Extrakte wurden mit 100 ml gesättigter Kochsalzlösung gewaschen und über Magnesumsulfat getrocknet. Nach Abdampfen der Lösungsmittel im Vakuum erhielt man 47,6 g Rohprodukt, das bei 0,002 mbar fraktioniert destilliert wurde. Die zwischen 120 und 130 °C siedende Fraktion war reines Produkt. Ausbeute: 38,3 g (78%, leicht gelbliches viskoses Öl).

IR (Film): ν = 3440, 3078, 2985, 2935, 2875, 2227, 1739, 1717, 1640, 1437, 1414, 1393, 1355, 1321, 1278, 1212, 1179, 1130, 1092, 1053, 1015, 927, 856, 779 cm⁻¹.

¹H-NMR (250 MHz, CDCl₃) : δ = 1,25 (t, ³*J* = 7,1 Hz, 3 H, CH₃), 1,96 (s, 1 H, OH), 2,16 (s, 3 H, CH₃), 2,70 (dddd, ²*J* = 14,3, ³*J* = 7,5, ⁴*J* = 1,2, 0,9 Hz, 1 H, =CHC*H₂*, *AB*-System), 2,77 (t, ⁵*J* = 2,1 Hz, 2 H, =CCH₂), 2,79 (dddd, ²*J* = 14,3, ³*J* = 7,5, ⁴*J* = 1,2, 0,9 Hz, 1 H, =CHC*H₂*, AB-System), 4,20 (t, ⁵*J* = 2,1 Hz, 2 H, =CCH₂O), 4,21 (q, ³*J* = 7,1 Hz, 2 H, OCH₂), 5,11 (ddt, ³*J* = 9,8, ²*J* = 1,9, ⁴*J* =0,9 Hz, 1 H, =CH₂), 5,16 (ddt, ³*J* = 17, ²*J* = 1,9, ⁴*J* = 1,2 Hz, 1 H, =CH₂), 5,55 (ddt, ³*J* = 17, 9,8, 7,5 Hz, 1 H, -CH=) ppm.

¹³C-NMR (62, 9 MHz, CDCl₃, DEPT) : δ = 14, 0 (CH₃), 21, 9 (CH₂), 26,5 (CH₃), 35,7 (CH₂), 51,0 (CH₂), 61,8 (CH₂), 62,6 (C), 80,5 (C), 81,8 (C), 199,8 (CH₂), 131,5 (CH₂), 170,4 4 (C), 202,8 (C) ppm.

MS (70 eV, DCI, NH₃) : *m*/*z* (%) = 494 (3) [2M + NH₄⁺], 256 (100) [M + NH₄⁺], 239 (2) [M + H⁺].

C₁₃H₁₈O₄ (238,28) : ber. C 65,53, H 7,61; gef. C 65.40, H 7.46.

### 2. Stufe: Kohlensäure-O-(5-acetyl-5-ethoxycarbonyl-oct-7-en-2-inyl)-O'-methylester

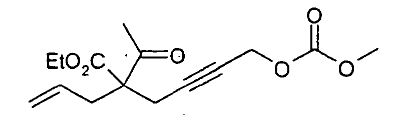

Zu einer gerührten Lösung von 2-Acetyl-2-allyl-6-hydroxy-hex-4-insäureethylester (38,1 g, 160 mmol), NEt₃ (23,3 ml, 167 mmol) und DMAP (0,98 g, 3 mmol) in 160 ml wasserfreiem Dichlormethan wurde bei -10 °C unter Stickstoff Chlorameisensäuremethylester (17 ml, 170 mmol, gelöst in 10 ml Dichlormethan) innerhalb einer Stunde getropft,wobei die Temperatur des Reaktionsgemisches unter -5 °C gehalten wurde. Dann wurde die Mischung noch für weitere 30 Minuten bei dieser Temperatur gerührt, das Kühlbad entfernt und der größte Teil des Lösungsmittels im Vakuum bei maximal Raumtemperatur entfernt. Der Rückstand wurde mit 80 ml Wasser und 200 ml Diethylether versetzt, die organische Phase abgetrennt und die wäßrige Phase noch zweimal mit je 50 ml Diethylether extrahiert. Die vereinigten organischen Phasen wurden über Magnesiumsulfat getrocknet. Nach dem Abziehen des Lösungsmittels erhielt man 47 g Rohprodukt, das durch Flash-Chromatographie gereinigt wurde (450 ml Flash-Kieselgel, Elutionsmittel: Pentan/Diethylether: 10:1 bis 3:1. Ausbeute: 45 g (95%, farbloses Öl).

IR (Film): **ν** = 3083, 2990, 2963, 2858, 2238, 2161, 2052, 1755, 1717, 1640, 1443, 1371, 1267, 1207, 1179, 1135, 1092, 1070, 1015, 1054, 954, 899, 856, 790 cm⁻¹.

¹H-NMR (250 MHz, CDCl₃) : δ = 1,25 (t, ³*J* = 7,1 Hz, 3 H, CH₃), 2,15 (s, 3 H, CH₃), 2,69 (dtdd, ²*J* = 15,0, ³*J* = 7,4, ⁴*J* = 1,2, 1,0 Hz, 1 H, 6-H, AB-System), 2,77 (t, ⁵*J* = 2,2 Hz, 2 H, 4-H), 2,79 (dtdd, ²*J* = 15,0, ³*J* = 7,4, ⁴*J* = 1,2, 1,0 Hz, 1 H, 6-H, *AB*-System), 3,79 (s, 3 H, CH₃), 4,20 (q, ³*J* = 7,1 Hz, 2 H, CH₂), 4,66 (t, ⁵*J* = 2,2 Hz, 2 H, 1-H), 5,10 (ddt, ³*J* = 9,7, ²*J* = 1,8, ⁴*J* = 1,0 Hz, 1 H, 8-H*ₜᵣₐₙₛ*), 5,15 (ddt, ³*J* = 17, 0, ²*J* = 1, 8, ⁴*J* = 1,2 Hz, 1 H, 8-H*_{cis}*), 5,51 (ddd, ³*J* = 17,0, ³*J* = 9,7 Hz, ³*J* = 7,4 Hz, 1 H, 7-H) ppm.

¹³C-NMR (62, 9 MHz, CDCl₃, DEPT): δ = 14, 0 (CH₃), 21, 9 (CH₂), 26,5 (CH₃), 35,8 (CH₂), 55,0 (CH₃), 55,7 (CH₂), 61,8 (CH₂), 62,5 (C), 76, 7 (C), 82,8 (C), 119,9 (CH₂), 131, 4 (CH), 155,1 (C), 170,2 (C), 202,5 (C) ppm.

MS (70 eV, EI) : *m*/*z* (%) = 296 (1) [M⁺], 259 (2), 257 (8), 254 (40) [M⁺ - C₂H₂O - ], 250 (6), 223 (7), 221 (41) [M⁺ - CH₃O - CO₂], 202 (14), 191 (9), 178 (100) [M⁺ - CH₃OH - C₂H₂O - CO₂], 175 (24), 169 (6), 163 (6), 149 (36), 147 (63) [M⁺ - C₂H₅O - CO₂ - CH₃OH - CO], 137 (13), 135 (8), 133 (16), 131 (14), 127 (4), 123 (9), 121 (7), 119 (6), 109 (7), 107 (8), 105 (46) [C₈H₉⁺], 103 (18) [C₈H₇⁺], 91 (13) [C₇H₇⁺], 79 (11) [C₆H₇⁺], 77 (14)

[C₆H₅⁺], 65 (2), 59 (4), 43 (42) [C₂H₃O⁺], 41 (2).

C₁₅H₂₀O₆ (296,32): ber. C 60,80, H 6,80; gef. C 61,16, H 6,77.

### 3. Stufe: 2-(3-Acetyl-3-ethoxycarbonyl-bicyclo[3.1.0]hex-1-yl)-acrylsäuremethylester

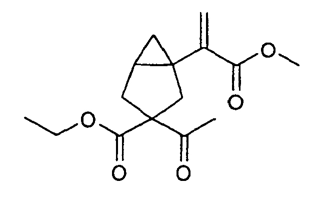

Zu einer Lösung von Tris(2-furyl)phosphan (1,581 g, 6,8 mmol) und Tetramethylammoniumbromid (527 mg, 3,4 mmol) in 800 ml entgastem Methanol gab man unter Argon Pd(OAc)₂ (694,4 mg, 3,1 mmol) und rührte das Gemisch für 1 h bei Raumtemperatur. Dann setzte man Kohlensäure-O-(5-acetyl-5-ethoxycarbonyl-oct-7-en-2-inyl)-O'-methylester (45 g, 152 mmol) hinzu und leitete Kohlenmonoxid ein. Das Gemisch wurde unter Kohlenmonoxid-Atmosphäre weiter kräftig bei 25 °C gerührt, bis das Ausgangsmaterial vollständig umgesetzt war (63 h, GC-Kontrolle). Das Methanol wurde im Vakuum abdestilliert, und man erhielt 45 g Rohprodukt, das durch Flash-Chromatographie (450 ml Flash-Kieselgel, Pentan/Diethylether, 8:1 bis 4:1) weiter gereinigt wurde. Ausbeute: 1. Fraktion: 4 g (Reinheit ca. 90%) und 35,3 g (83%, GC: 98%, schwach gelbliches Öl. Abschließend wurde eine Kugelrohrdestillation (140 °C; 0,001 mbar) durchgeführt. Ausbeute: 30,6 g (72%; GC:98,5%, farbloses Öl).

IR (Film) : ν = 3078, 2985, 2952, 2875, 1717, 1624, 1437, 1355, 1289, 1234, 1212, 1163, 1124, 1092, 1070, 1015, 993, 954, 856, 812, 773, 691 cm⁻¹.

Hauptisomer: ¹H-NMR (250 MHz, CDCl₃) : δ = 0,35 (dd, ²*J* = 5,6, ³*J* = 4,5 Hz, 1 H, 6-H*_{endo}*), 0,67 (dddd, ³*J* = 7,8, ²*J* = 5,6, ⁴*J* = 1,5, ⁴*J* = 1,5 Hz, 1 H, 6-H*ₑₓₒ*), 1,22 (t, ³*J* = 7,1 Hz, 3 H, CH₃), 1,48-1,55 (m, 1 H, 5-H), 2,15 (s, 3 H, CH₃), 2,40-2,70 [m, 4 H, 2(4)-H], 3,75 (s, 3 H, CH₃), 4,15 (q, ³*J* = 7,1 Hz, 2 H, CH₂), 5,59 (d, ²*J* = 1, 4 Hz, 1 H, =CH₂), 6, 15 (d, ²*J* = 1,4 Hz, 1 H, =CH₂) ppm.

¹³C-NMR (62, 9 MHz, CDCl₃, DEPT) : δ = 14, 0 (CH₃), 16, 4 (CH₂), 24,8 (CH), 26,6 (CH), 31,2 (C), 34,5 (CH₂), 38,8 (CH₂), 51, 8 (CH₃), 61,8 (CH₂), 67,0 (C), 125,8 (CH₂), 142,1 (C), 167,0 (C), 171,9 (C), 204,6 (C) ppm.

Nebenisomer: ¹H-NMR (250 MHz, CDCl₃) : δ = 0,49 (dd, ²*J* = 5,6, ³*J* = 4,5 Hz, 1 H, 6-H*_{endo}*), 0,76 (dddd, ³*J* = 7,8, ²*J* = 5,6, ⁴*J* = 1,5, ⁴*J* = 1,5 Hz, 1 H, 6-H*ₑₓₒ*), 1,26 (t, ³*J* = 7,1 Hz, 3 H, CH₃), 1,47-1,54 (m, 1 H, 5-H), 2,13 (s, 3 H, CH₃), 2,40-2,70 [m, 4 H, 2(4)-H], 3,74 (s, 3 H, CH₃), 4,19 (q, ³*J* = 7,1 Hz, 2 H, CH₂), 5,61 (d, ²*J* = 1,4 Hz, 1 H, =CH₂), 6,16 (d, ²*J* = 1,4 Hz, 1 H, =CH₂) ppm.

¹³C-NMR (62, 9 MHz, CDCl₃, DEPT) : δ = 14, 0 (CH₃), 16, 4 (CH₂), 24,9 (CH), 25,9 (CH), 31,1 (C), 34,5 (CH₂), 38,6 (CH₂), 51,8 (CH₃), 61,8 (CH₂), 67,1 (C), 126,1 (CH₂), 142,0 (C), 166,9 (C), 173,4 (C), 202, 1 (C) ppm.

MS (70 eV, EI) : *m*/*z* (%) = 280 (12) [M⁺], 248 (100) [M⁺ - CH₃O - H], 237 (20) [M⁺ - CH₃CO], 234 (56) [M⁺ - C₂H₅O - H], 220 (37) [M⁺ - CH₃O - CO - H], 206 (19) [M⁺ - C₂H₅O - CO - H], 191 (61) [M⁺ - CH₃O - C₂H₅ - CO - H], 177 (70) [M⁺ - CH₃CO - CH₃O - CO - H], 175 (28), 163 (18) [M⁺ - C₂H₅O - CH₃CO - CO - H], 159 (16), 154 (16), 149 (34), 146 (78) [M⁺ - C₂H₅O - CH₃O - 2 CO - 2 H], 131 (55) [M⁺ - C₂H₅O - CH₃CO - CH₃O - CO - 2 H], 119 (8), 105 (61) [C₈H₉⁺], 103 (84) [C₈H₇⁺], 91 (19) [C₇H₇⁺], 79 (18), 77 (24) [C₆H₅⁺], 59 (5), 55 (5), 43 (94) [C₂H₃O⁺].

C₁₅H₂₀O₅ (280, 32) : ber. C 64,27, H 7,19; gef. C 63,92, H 7,12.

### Beispiel 3: Radikalische Lösungspolymerisation des funktionalisierten Cyclopropylacrylats ABHCE aus Beispiel 2

In einem Schlenkgefäss wurden zu einer Lösung des Cyclopropylacrylats aus Beispiel 2 (ABHCE) (2,0 mol/l) in Chlorbenzol 2,0 mol-% (bezogen auf das Monomer) Azobisisobutyronitril (AIBN) gegeben. Nach Entgasen der Monomerlösung und Verschliessen des Schlenkgefässes unter Argon wurde im thermostatierten Wasserbad bei 65 °C polymerisiert. Nach 1 bzw. 15 h wurde die Polymerisation durch Ausfällen des Polymerisats mit der zehnfachen Menge Hexan abgebrochen. Das gebildete Polymer wurde abfiltriert und bis zur Gewichtskonstanz getrocknet. Die Ausbeute betrug nahezu 58 bzw. 95 % an einem weißen Homopolymeren mit einer zahlenmittleren Molmasse von 296.000 bzw. 434.400 g/mol und einer Glasübergangstemperatur von 90 °C bzw. 97 °C. Die ¹H-und ¹³C-NMR-Spektren der gebildeten Polymeren belegen, daß die Polymerisation von ABHCE unter Öffnung des Cyclopropan-Ringes verlaufen ist.

### Beispiel 4: Radikalische Copolymerisation des funktionalisierten Cyclopropylacrylats aus Beispiel 2 (ABHCE) mit Methylmethacrylat (MMA)

Analog zur Homopolymerisation in Lösung (Beispiel 3) wurde eine Monomermischung des Cyclopropylacrylats aus Beispiel 2 (ABHCE) (1,0 mol/l), Methylmethacrylat (MMA, 1,0 mol/l) und AIBN (2,5 mol-%) in Chlorbenzol hergestellt und polymerisiert. Die Ausbeute an Copolymer betrug nach 15 Minuten 5,4 %. Dabei wurde ¹H-NMR-spektroskopisch eine molare Copolymerzusammensetzung von ABHCE: MMA = 1,00 : 1,13 ermittelt. Dieses Ergebnis belegt eine mit dem Methacrylat MMA vergleichbare Reaktivität des Cyclopropylacrylates ABHCE.

### Beispiel 5: Radikalische Copolymerisation des funktionalisierten Cyclopropylacrylats aus Beispiel 2 (ABHCE) mit UDMA

Zur Bestimmung des Polymerisationsschrumpfes wurde eine Mischung aus 50 Gew.-% des Cyclopropylacrylats aus Beispiel 2 (ABHCE) und 50 Gew.-% UDMA (Urethandimethacrylat aus 2 Mol 2-Hydroxyethylmethacrylat und 1 Mol 2,2,4-Trimethylhexamethylendiisocyanat) hergestellt, mit 0,3 Gew. (bezogen auf die Gesamtmischung) Campherchinon (Photointiator) und 0,5 Gew.-% 4-(Dimethylamino)-benzoesäureethylester (Aminbeschleuniger) versetzt und anschließend mit einer dentalen Lichtquelle (Spectramat, Ivoclar Vivadent AG) bestrahlt. Aus der Differenz der bestimmten Dichten der Monomermischung bzw. des gebildeten Polymerisates wurde unter Berücksichtigung des Polymerisationsschrumpfes von reinem UDMA (ΔV_{P} = 6,1 %) ein Polymerisationsschrumpf von nur 5,1 % berechnet. Aus den Ergebnissen der Polymerisation einer analogen Mischung von MMA und UDMA (50 : 50) wurde der für MMA bekannte Polymerisationsschrumpf von 20,7 % berechnet.

Beispiele 4 und 5 zeigen, daß sich die erfindungsgemäßen funktionalisierten Cyclopropylacrylate durch ähnliche Reaktivität wie Methacrylate auszeichnen, aber im Gegensatz zu Methacrylaten einen sehr geringen Polymerisationsschrumpf ergeben.

### Beispiel 6: Herstellung eines Dentalzementes auf der Basis des Cyclopropylacrylats aus Beispiel 2 (ABHCE)

Entsprechend der nachfolgend aufgeführten Tabelle 1 wurde ein Kompositbefestigungszement auf der Basis von A einer Methacrylatmischung (Vergleich) und B des Monomers ABHCE aus Beispiel 2 mittels eines Walzenstuhles "Exakt" (Exakt Apparatebau, Norderstedt) hergestellt. Von den Materialien wurden entsprechende Prüfkörper mit den Abmessungen 2x2x25 mm präpariert, die durch zweimaliges Bestrahlen für je 3 Minuten mit einer dentalen Lichtquelle (Spectramat, Ivoclar Vivadent AG) gehärtet wurden. Von den gehärteten Prüfkörpern wurden anschließend die mechanischen Eigenschaften nach der ISO-Norm 4049 ermittelt.

Aus Tabelle 2 ist ersichtlich, daß das gehärtete erfindungsgemäße Material B in seinen mechanischen Eigenschaften in jeder Hinsicht dem Vergleichsmaterial A entsprich.

Das Beispiel zeigt, daß Dentalmaterialien auf der Basis von erfindungsgemäßen funktionellen Cyclopropylacrylaten wie ABHCE trotz einer ähnlichen Reaktivität und eines stark verringerten Polymerisationschrumpfes keine Nachteile im Hinblick auf die mechanischen Eigenschaften aufweisen.

**Tabelle 1: Zusammensetzung der Zemente**

| **Stoff** | **Material A¹⁾ Anteile (Gew.-%)** | **Material B Anteile (Gew.-%)** |
|---|---|---|
| UDMA | 31,6 | 31,6 |
| 1,10-Decandioldimethacrylat | 7,8 | - |
| Monomer aus Bsp. 2 (ABHCE) | - | 7,8 |
| Aerosil OX-50(Degussa) | 41,3 | 41,3 |
| Ytterbiumtrifluorid (Rhone-Poulenc) | 18,7 | 18,7 |
| Photoinitiator²) | 0,5 | 0,5 |

| | | |
|---|---|---|
| ¹⁾ Vergleichsbeispiel ²⁾ 1:1-Mischung aus Campherchinon und p-N,N-Dimethylaminobenzoesäureethylester | | |

**Tabelle 2: Mechanische Eigenschaften der Zemente**

| **Materialeigenschaft** | **Material A¹⁾** | **Material B** |
|---|---|---|
| Biegefestigkeit (MPa) nach 24 h | 95 | 99 |
| Biegefestigkeit (MPa) nach 24 h WL²⁾ | 101 | 99 |
| Biegefestigkeit (MPa) nach 7 d WL | 111 | 108 |
| Biege-E-Modul (GPa) nach 24 h | 4,76 | 4,78 |
| Biege-E-Modul (GPa) nach 24 h WL | 4,93 | 4,56 |
| Biege-E-Modul (GPa) nach 7 d WL | 5,13 | 4,70 |

| | | |
|---|---|---|
| ¹⁾ Vergleichsbeispiel ²⁾ WL = Wasserlagerung der Prüfkörper bei 37 °C | | |

### Beispiel 7: Herstellung eines Füllungskomposits auf Basis des Cyclopropylacrylats aus Beispiel 2 (ABHCE)

Entsprechend der nachfolgend aufgeführten Tabelle 3 wurde ein Füllungskomposit D auf der Basis einer Methacrylatmischung und des Monomers ABHCE aus Beispiel 2 mittels eines Laborkneters LPM 0.1 (Fa. Linden, Marienheide) hergestellt. Von den Materialien wurden Prüfkörper mit den Abmessungen 2x2x25 mm präpariert, die durch zweimaliges Bestrahlen für je 3 Minuten mit einer dentalen Lichtquelle (Spectramat, Ivoclar Vivadent AG) gehärtet wurden. Von den gehärteten Prüfkörpern wurden anschließend die mechanischen Eigenschaften nach der ISO-Norm 4049 ermittelt.

Der dilatometrisch ermittelte Polymerisationsschrumpf betrug nur 2,2 % während für ein Vergleichskomposit C, bei dem das erfindungsgemäße Monomer ABHCE durch den üblichen Dimethacrylatverdünner 1,10-Decandioldimethacrylat (D₃MA) ersetzt wurde, ein Polymerisationsschrumpf von 3,2 % gemessen wurde.

Das Beispiel zeigt, daß Füllungsmaterialien auf der Basis von erfindungsgemäßen Cyclopropylacrylaten wie ABHCE gute mechanische Eigenschaften aufweisen.

**Tabelle 3: Zusammensetzung der Füllungsmaterialen**

| **Stoff** | **Anteile (Gew.-%)** | |
|---|---|---|
| | **Komposit C¹⁾** | **Komposit D** |
| SR-348C (Sartomer)²⁾ | 7,2 | 7,2 |
| 1,10-Decandioldimethacrylat | 10,3 | - |
| ABHCE | - | 10,3 |
| Glasfüller GM27884³⁾ | 51,2 | 51,2 |
| Aerosil OX-50 (Degussa) | 1,0 | 1,0 |
| Ytterbiumtrifluorid (Rhone-Poulenc) | 14,9 | 14,9 |
| Sphärosil⁴⁾ | 14,2 | 14,2 |
| Photoinitiator⁵⁾ | 0,2 | 0,2 |

| | | |
|---|---|---|
| ¹⁾ Vergleichsbeispiel ²⁾ Ethoxyliertes Bisphenol-A-dimethacrylat mit insgesamt 3 Ethylenoxideinheiten ³⁾ Bariumaluminiumsilikatglas (Schott) silanisiert, mittlere Partikelgrösse 1,2 µm ⁴⁾ SiO₂-ZrO₂-Mischoxid mit einer Primärpartikelgrösse von ca. 200 nm (Tokoyama Soda) ⁵⁾ Mischung aus Campherchinon (0,05 %), p-N,N-Dimethylaminoben-zoesäureethylester (0,08 %) und Lucirin TPO (0,07 %, BASF) | | |

**Tabelle 4: Mechanische Eigenschaften der Füllungsmaterialen**

| **Materialeigenschaft** | **Komposit C¹⁾** | **Komposit D** |
|---|---|---|
| Biegefestigkeit (MPa) nach 24 h | 144 | 112 |
| Biegefestigkeit (MPa) nach 24 h WL²⁾ | 145 | 120 |
| Biegefestigkeit (MPa) nach 7 d WL | 140 | 112 |
| Biege-E-Modul (GPa) nach 24 h | 9,55 | 9,65 |
| Biege-E-Modul (GPa) nach 24 h WL | 9,24 | 9,30 |
| Biege-E-Modul (GPa) nach 7 d WL | 9,18 | 9,70 |

| | | |
|---|---|---|
| ¹⁾ Vergleichsbeispiel ²⁾ WL = Wasserlagerung der Prüfkörper bei 37 °C | | |

## Patentansprüche

1. Zusammensetzung, die
(a) ein Cyclopropylacrylat der allgemeinen Formel (1), 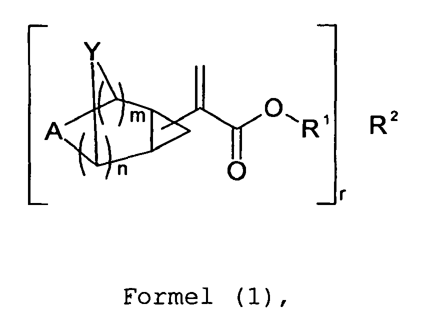 in der A ausgewählt ist aus 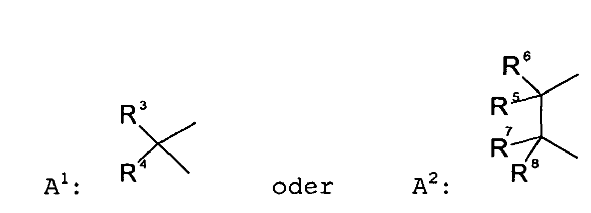 und
und die übrigen Variablen unabhängig voneinander die folgenden Bedeutungen haben:
Y = für A = A¹: entfällt, CH₂ oder O,
für A = A²: CH₂ oder O,
n = für Y entfällt: 0, 1, 2 oder 3,
für Y = CH₂ oder O: 1,
m = für Y entfällt: 0, 1, 2 oder 3,
für Y = CH₂ oder O: 1,
r = 1, 2, 3 oder 4,
R¹ = H, ein C₁- bis C₁₀-Alkyl-, C₆- bis C₁₂-Aryl-, C₁- bis C₁₀-Arylalkyl- oder bicyclischer C₅-C₁₂-Rest oder -R¹⁰-X-,
R² = ein durch den Klammerausdruck r-fach substituier- ter aliphatischer C₁-C₂₀-Kohlenwasserstoffrest, der durch O oder S unterbrochen sein kann, cycloali- phatischer C₄-C₁₂-Rest, bicyclischer C₅-C₁₂-Rest, C₆-C₁₄-Aryl- oder C₇-C₂₀-Alkylaryl-Rest,
R³ = -CO-OR⁹, -CO-R⁹, -S(O)R⁹, -SO₂R⁹, -SO₂(OR⁹), -PO(OR⁹)₂, -CN, -H, -R⁹,
R⁴ = -CO-OR⁹, -CO-R⁹, -S(O)R⁹, -SO₂R⁹, -SO₂(OR), -PO(OR⁹)₂, -CN, oder -R¹¹-R¹⁰-X-
R⁵-R⁸ = unabhängig voneinander H, -CO-OR⁹, -CO-NHR⁹, -CO- NR⁹₂, -CO-R⁹, -CN, ein C₁-C₂₀-Alkyl-Rest, der durch O oder S unterbrochen sein kann, ein cycloalipha- tischer C₄-C₁₂-Rest, ein bicyclischer C₅-C₁₂-Rest, ein C₆-C₁₄-Aryl-, C₇-C₂₀-Alkylaryl-Rest, oder minde- stens zwei der Reste bilden zusammen mit den Koh- lenstoffatomen, an die sie gebunden sind, ein 5- bis 8-gliedriges Ringsystem,
R⁹ = ein C₁-C₂₀-Alkyl-Rest, der durch O oder S unterbro- chen sein kann, ein cycloaliphatischer C₄-C₁₂-Rest, ein bicyclischer C₅-C₁₂-Rest, ein C₆-C₁₄₋Aryl-, oder C₇-C₂₀-Alkylaryl-Rest,
R¹⁰ = entfällt oder ein C₁-C₂₀-Alkylen-Rest, der durch O oder S unterbrochen sein kann, ein cycloaliphati- scher bzw. bicyclischer C₅-C₁₂-Rest, ein C₆₋C₁₄- Arylen- oder C₇-C₂₀-Alkylenarylen-Rest,
R¹¹ = -COO-, -CO-, -SO-, -SO₂-, -SO₂(O-), -PO(OR⁹)(O-),
X = entfällt, -O-CO-, -CO-O-, -NH-CO-, -CO-NH-, -NH-CO-O- oder -O-CO-NH-, wobei X die Be- deutung "entfällt" hat, wenn R¹⁰ entfällt,
wobei die Reste R² und R⁵⁻⁹ substituiert oder unsubstituiert sein können, entweder R⁴ = -R¹¹-R¹⁰-X- oder R¹ = -R¹⁰-X- ist und der Klammerausdruck über X an R² gebunden ist und wobei mehrere Reste eines Typs gleich oder verschieden sein können und die Substituenten der Reste R² und R⁵⁻⁹ ggf. ausgewählt sind aus Alkyl, Halogen, OCH₃, OC₂H₅, Vinyl, (Meth) acryl, COOR¹², SiCl₃, Si(OR¹³)₃, und mesogenen Gruppen, wobei
R¹² = H oder C₁- bis C₁₀-Alkyl-, C₆- bis C₁₂-Aryl-, C₆- bis C₁₀-Arylalkyl- oder ein bicyclischer C₅-C₁₂₋ Rest und
R¹³ = H oder ein C₁- bis C₁₀-Alkyl-Rest,
ein Stereoisomer oder eine Mischung davon sowie
(b) einen Initiator für die radikalische Polymerisation enthält.

2. Zusammensetzung nach Anspruch 1, bei dem A = A¹ und R¹ = -R¹⁰-X- ist und Y entfällt.

3. Zusammensetzung nach Anspruch 1, bei dem A = A¹ und R⁴ = -R¹¹-R¹⁰-X- ist und Y entfällt.

4. Zusammensetzung nach Anspruch 1, bei dem A = A², R¹ = -R¹⁰-X-und Y CH₂ oder O ist.

5. Zusammensetzung nach einem der Ansprüche 1 bis 4, bei dem mindestens eine Variable der Formel (1) eine der folgenden Bedeutungen hat:
Y = für A = A¹: entfällt, für A = A²: CH₂ oder O,
n = 1,
m = 1,
r = 1 oder 2,
R¹ = H, C₁- bis C₅-Alkyl- oder bicyclischer C₅-C₁₂₋Rest oder insbesondere -R¹⁰-X-,
R² = ein aliphatischer C₁-C₆-Kohlenwasserstoffrest, der durch O unterbrochen sein kann, ein cycloaliphati- scher C₆-C₈-Rest, ein bicyclischer C₆-C₈-Rest, ein C₆-C₁₀-Aryl- oder C₇-C₁₀-Alkylaryl-Rest,
R³ = -CO-OR⁹, CO-R⁹, -SO₂R⁹, CN, H oder -R⁹,
R⁴ = -CO-OR⁹, -CO-R⁹, -SO₂R⁹, CN oder -R¹¹-¹⁰-X-
R⁵-R⁸ = unabhängig voneinander H, -CO-OR⁹, -CO-R⁹, CN, ein C₁-C₆-Alkyl-Rest, der durch O unterbrochen sein kann, ein cycloaliphatischer C₆-C₈-Rest, ein bicy- clischer C₆-C₈-Rest, ein C₆-C₁₀-Aryl- oder C₇-C₁₀- Alkylaryl-Rest,
R⁹ = ein C₁-C₆-Alkyl-Rest, der durch O unterbrochen sein kann, ein cycloaliphatischer C₆-C₈-Rest, ein bicyclischer C₆-C₈-Rest, ein C₆-C₁₀-Aryl- oder C₇- C₁₀-Alkylaryl-Rest, insbesondere C₁-C₃-Alkyl,
R¹⁰ = entfällt oder ein C₁-C₁₀-Alkylen-Rest, der durch O unterbrochen sein kann, ein bicyclischer C₆-C₉- Rest, oder C₇-C₁₀-Alkylenarylen-Rest, insbesondere entfällt oder C₁-C₆-Alkylen,
R¹¹ = -CO-O-, -CO- oder -SO₂-, insbesondere -CO-O-,
X = entfällt, -O-CO- oder -CO-O-.

6. Zusammensetzung nach einem der Ansprüche 1 bis 5, die zusätzlich mindestens ein weiteres radikalisch polymerisierbares Monomer enthält.

7. Zusammensetzung nach Anspruch 6, die als zusätzlich radikalisch polymerisierbares Monomer ein mehrfunktionelles Monomer enthält.

8. Zusammensetzung nach Anspruch 7, die als mehrfunktionelles radikalisch polymerisierbares Monomer ein bi- oder mehrfunktionelles Acrylat oder Methacrylat, Bisphenol-A-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat und 2,2,4-Trimethylhexamethylendiisocyanat), Di-, Tri- oder Tetraethylenglycoldi(meth)acrylat, Trimethylolpropantri(meth)acrylat, Pentaerythrittetra(meth)acrylat, Butandioldi(meth)acrylat, 1,10-Decandioldi(meth)acrylat oder 1,12-Dodecandioldi(meth)acrylat, ein Urethan aus 2-(Hydroxymethyl)acrylsäure und Diisocyanten, wie 2,2,4-Trimethylhexamethylendiisocyanat oder Isophorondiisocyanat, ein vernetzendes Pyrrolidon, wie 1,6-Bis(3-vinyl-2-pyrolidonyl)-hexan; ein Bisacrylamid, Methylen- oder Ethylenbisacrylamid, Bis(meth)acrylamid, N,N'-Diethyl-1,3-bis(acrylamido)-propan, 1,3-Bis(methacrylamido)-propan, 1,4-Bis(acrylamido)-butan, N,N'-Bis-(acryloyl)-piperazin, oder eine Mischung von zwei oder mehr dieser Monomere enthält.

9. Zusammensetzung nach einem der Ansprüche 1 bis 8, die zusätzlich Füllstoff enthält.

10. Zusammensetzung nach einem der Ansprüche 1 bis 9, die
a) 1 bis 80 Gew.-% Cyclopropylacrylat gemäß Formel (1),
b) 0,01 bis 5 Gew.-% Initiator für die radikalische Polymerisation,
c) 0 bis 60 Gew.-% radikalisch polymerisierbares Monomer,
d) 0 bis 40 Gew.-% Lösungsmittel und
e) 0 bis 20 Gew.-% Füllstoff
enthält.

11. Zusammensetzung nach einem der Ansprüche 1 bis 9, die
a) 1 bis 60 Gew.-% Cyclopropylacrylat gemäß Formel (1),
b) 0,01 bis 5 Gew.-% Initiator für die radikalische Polymerisation,
c) 0 bis 60 Gew.-% radikalisch polymerisierbares Monomer und
d) 20 bis 60 Gew.-% Füllstoff
enthält.

12. Zusammensetzung nach einem der Ansprüche 1 bis 9, die
a) 1 bis 45 Gew.-% Cyclopropylacrylat gemäß Formel (1),
b) 0,01 bis 5 Gew.-% Initiator für die radikalische Polymerisation,
c) 0 bis 50 Gew.-% radikalisch polymerisierbares Monomer und
d) 30 bis 85 Gew.-% Füllstoff
enthält..

13. Zusammensetzung nach einem der Ansprüche 1 bis 9, die
a) 1 bis 95 Gew.-% Cyclopropylacrylat gemäß Formel (1),
b) 0,01 bis 5 Gew.-% Initiator für die radikalische Polymerisation,
c) 0 bis 60 Gew.-% radikalisch polymerisierbares Monomer und
d) 0 bis 20 Gew.-% Füllstoff
enthält.

14. Zusammensetzung nach einem der Ansprüche 1 bis 13, die zusätzlich mindestens einen weiteren Bestandteil ausgewählt aus der Gruppe bestehend aus Stabilisatoren, UV-Absorbern, Farbstoffen, Pigmenten und Gleitmitteln enthält.

15. Zusammensetzung nach einem der Ansprüche 1 bis 14 in gehärteter Form.

16. Verwendung eines Cyclopropylacrylats gemäß Formel (1) zur Herstellung eines Dentalmaterials.

17. Verwendung einer Zusammensetzung gemäß einem der Ansprüche 1 bis 15 als Dentalmaterial.

18. Verwendung eines Cyclopropylacrylats oder einer Zusammensetzung nach Anspruch 16 oder 17, wobei das Dentalmaterial ein Adhäsiv ist.

19. Verwendung eines Cyclopropylacrylats oder einer Zusammensetzung nach Anspruch 16 oder 17, wobei das Dentalmaterial ein Zement ist.

20. Verwendung eines Cyclopropylacrylats oder einer Zusammensetzung nach Anspruch 16 oder 17, wobei das Dentalmaterial ein Füllmaterial ist.

21. Verwendung eines Cyclopropylacrylats oder einer Zusammensetzung nach Anspruch 16 oder 17, wobei das Dentalmaterial ein Beschichtungsmaterial ist.

## Claims

1. A composition that comprises
(a) a cyclopropyl acrylate of the general Formula (1), 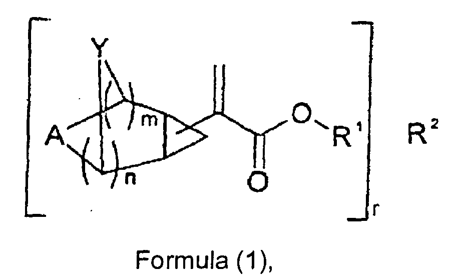 in which A is selected from 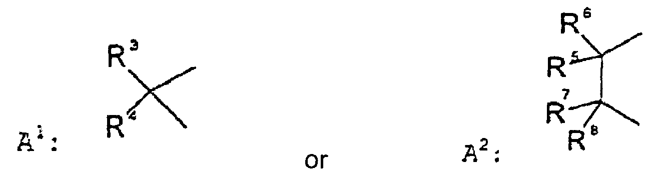 and the remaining variables have the following meanings independently of each other:
Y = for A = A¹: is absent, CH₂ or O,
for A = A²: CH₂ or O,
n = for Y is absent: 0, 1, 2 or 3,
for Y = CH₂ or O: 1,
m = for Y is absent: 0, 1, 2 or 3,
for Y = CH₂ or O: 1,
r = 1, 2, 3 or 4,
R¹ = H, a C₁ to C₁₀ alkyl, C₆ to C₁₂ aryl, C₁ to C₁₀ arylalkyl or bicyclic C₅-C₁₂ group or -R¹⁰-X-,
R² = an aliphatic C₁-C₂₀ hydrocarbon group, substituted r times by the expression in brackets, which can be interrupted by O or S, cycloaliphatic C₄-C₁₂ group, bicyclic C₅-C₁₂ group, C₆-C₁₄ aryl or C₇-C₂₀ alkylaryl group,
R³ = -CO-OR⁹, -CO-R⁹, -S(O)R⁹, SO2R⁹, -SO2(OR⁹), - PO(OR⁹)₂, -CN, -H, -R⁹,
R⁴ = -CO-OR⁹, -CO-R⁹, -S(O)R⁹, SO₂R⁹, -SO₂(OR⁹), -PO(OR⁹)₂, -CN, or -R¹¹-R¹⁰-X-,
R⁵ -R⁸ = independently of one another H, -CO-OR⁹, -CO-NHR⁹, - CO-NR⁹₂, -CO-R⁹, -CN, a C₁-C₂₀ alkyl group that can be interrupted by O or S, a cycloaliphatic C₄-C₁₂ group, a bicyclic C₅-C₁₂ group, a C₆-C₁₄ aryl group, C₇-C₂₀ alkylaryl group, or at least two of the groups form, together with the carbon atoms to which they are bonded, a 5- to 8- membered ring system,
R⁹ = a C₁-C₂₀ alkyl group that can be interrupted by O or S, a cycloaliphatic C₄-C₁₂ group, a bicyclic C₅-C₁₂ group, a C₆- C₁₄ aryl group or C₇-C₂₀ alkylaryl group,
R¹⁰ = is absent or a C₁-C₂₀ alkylene group that can be interrupted by O or S, a cycloaliphatic or bicyclic C₅-C₁₂ group, a C₆- C₁₄ arylene group or C₇-C₂₀ alkylenearylene group,
R¹¹ = -COO-, -CO-, -SO-, -SO₂-, -SO₂(O-), -PO(OR⁹)(O-),
X = is absent, -O-CO-, -CO-O-, -NH-CO-, -CO-NH-, -NH-CO- O- or -O-CO-NH-, wherein X has the meaning "is absent" if R¹⁰ is absent, and
wherein the groups R² and R⁵⁻⁹ can be substituted or unsubstituted, either R⁴ = -R¹¹-R¹⁰-X- or R¹ = -R¹⁰-X- and the expression in brackets is bonded to R² through X and wherein a plurality of groups of one type can be identical or different and the substituents of the groups R² and R⁵⁻⁹ are optionally selected from alkyl, halogen, OCH₃, OC₂H₅, vinyl, (meth)acryl, COOR¹², SiCl₃, Si(OR¹³)₃, and mesogenic groups, wherein
R¹² = H or C₁ to C₁₀ alkyl, C₆ to C₁₂ aryl, C₆ to C₁₀ arylalkyl or a bicyclic C₅-C₁₂ group and
R¹³ = H or a C₁ to C₁₀ alkyl group,
a stereoisomer or a mixture thereof as well as
(b) an initiator for the radical polymerisation.

2. The composition according to Claim 1, in which A = A¹ and R¹ = -R¹⁰-X-and Y is absent.

3. The composition according to Claim 1 in which A = A¹ and R⁴ = -R¹¹-R¹⁰-X- and Y is absent.

4. The composition according to claim 1, in which A = A², R¹ = -R¹⁰-X- and Y is CH₂ or O.

5. The composition according to one of Claims 1 to 4, in which at least one variable of Formula (1) has one of the following meanings:
Y = for A = A¹: is absent,
for A = A²: CH₂ or O,
n = 1,
m = 1,
r = 1 or 2,
R¹ = H, C₁ to C₅ alkyl or bicyclic C₅-C₁₂ group or in particular - R¹⁰-X-,
R² = an aliphatic C₁-C₆ hydrocarbon group that can be interrupted by O, a cycloaliphatic C₆-C₈ group, a bicyclic C₆-C₈ group, a C₆-C₁₀ aryl or C₇-C₁₀ alkylaryl group,
R³ = -CO-OR⁹, -CO-R⁹, SO₂R⁹, CN, H or -R⁹,
R⁴ = -CO-OR⁹, -CO-R⁹, SO₂R⁹, CN or -R¹¹-R¹⁰-X-,
R⁵-R⁸ = independently of one another H, -CO-OR⁹, -CO-R⁹, CN, a C₁-C₆ alkyl group that can be interrupted by O, a cycloaliphatic C₆-C₈ group, a bicyclic C₆-C₈ group, a C₆-C₁₀ aryl or C₇-C₁₀ alkylaryl group,
R⁹ = a C₁-C₆ alkyl group that can be interrupted by O, a cycloaliphatic C₆-C₈ group, a bicyclic C₆-C₈ group, a C₆-C₁₀ aryl or C₇-C₁₀ alkylaryl group, especially C₁-C₃ alkyl,
R¹⁰ = is absent or a C₁-C₁₀ alkylene group that can be interrupted by O, a bicyclic C₆-C₉ group, or C₇-C₁₀ alkylenearylene group, especially is absent or C₁-C₆ alkylene,
R¹¹ = -CO-O-, -CO- or -SO₂-, especially -CO-O-,
X = is absent, -O-CO- or -CO-O-.

6. The composition according to one of Claims 1 to 5 which additionally comprises at least one additional radically polymerisable monomer.

7. The composition according to Claim 6 which comprises a polyfunctional monomer as the additional radically polymerisable monomer.

8. The composition according to Claim 7 which comprises a bi- or polyfunctional acrylate or methacrylate, bisphenol-A-di(meth)acrylate, bis-GMA (an addition product of methacrylic acid and bisphenol-A-diglycidyl ether), UDMA (an addition product of 2-hydroxyethyl methacrylate and 2,2,4-trimethylhexamethylene diisocyanate), di-, tri- or tetraethylene glycol di(meth)acrylate, trimethylolpropane tri(meth)acrylate, pentaerythritol tetra(meth)acrylate, butane diol di(meth)acrylate, 1,10-decane diol di(meth)acrylate or 1,12-dodecane diol di(meth)acrylate, a urethane of 2-(hydroxymethyl)acrylic acid and diisocyanates, such as 2,2,4-trimethylhexamethylene diisocyanate or isophorone diisocyanate, a cross-linking pyrrolidone, such as 1,6-bis(3-vinyl-2-pyrrolidonyl)-hexane, a bisacrylamide, methylene or ethylene bisacrylamide, bis(meth)acrylamide, N,N'-diethyl-1,3-bis(acrylamido)-propane, 1,3-bis(methacrylamido)-propane, 1,4-bis(acrylamido)-butane, N,N'-bis-(acryloyl)-piperazine, or a mixture of two or more of these monomers, as the polyfunctional radically polymerisable monomer.

9. The composition according to one of Claims 1 to 8 which additionally comprises filler.

10. The composition according to one of Claims 1 to 9 which comprises
a) 1 to 80 wt. % cyclopropyl acrylate according to Formula (1),
b) 0.01 to 5 wt. % initiator for the radical polymerisation,
c) 0 to 60 wt. % radically polymerisable monomer,
d) 0 to 40 wt. % solvent and
e) 0 to 20 wt. % filler.

11. The composition according to one of Claims 1 to 9 which comprises
a) 1 to 60 wt. % cyclopropyl acrylate according to Formula (1),
b) 0.01 to 5 wt. % initiator for the radical polymerisation,
c) 0 to 60 wt. % radically polymerisable monomer and
d) 20 to 60 wt. % filler.

12. The composition according to one of Claims 1 to 9 which comprises
a) 1 to 45 wt. % cyclopropyl acrylate according to Formula (1),
b) 0.01 to 5 wt. % initiator for the radical polymerisation,
c) 0 to 50 wt. % radically polymerisable monomer and
d) 30 to 85 wt. % filler.

13. The composition according to one of Claims 1 to 9 which comprises
a) 1 to 95 wt. % cyclopropyl acrylate according to Formula (1),
b) 0.01 to 5 wt. % initiator for the radical polymerisation,
c) 0 to 60 wt. % radically polymerisable monomer and
d) 0 to 20 wt. % filler.

14. The composition according to one of Claims 1 to 13 which additionally comprises at least one further constituent selected from the group consisting of stabilisers, UV absorbers, colorants, pigments and lubricants.

15. The composition according to one of Claims 1 to 14 in cured form.

16. The use of a cyclopropyl acrylate according to Formula (1) for preparing a dental material.

17. The use of a composition according to one of Claims 1 to 15 as a dental material.

18. The use of a cyclopropyl acrylate or a composition according to Claim 16 or 17 wherein the dental material is an adhesive.

19. The use of a cyclopropyl acrylate or of a composition according to Claim 16 or 17 wherein the dental material is a cement.

20. The use of a cyclopropyl acrylate or a composition according to Claim 16 or 17 wherein the dental material is a filling material.

21. The use of a cyclopropyl acrylate or a composition according to Claim 16 or 17, wherein the dental material is a coating material.

## Revendications

1. Composition qui contient :
a) un cyclopropyl-acrylate de formule générale (1) : 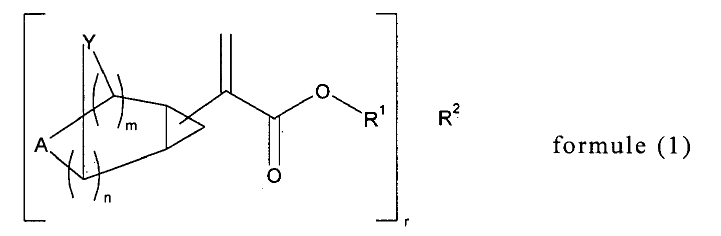 dans laquelle A représente un groupe choisi parmi les groupes 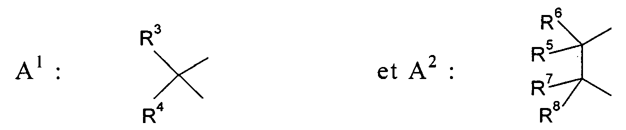 et les autres symboles ont, indépendamment les uns des autres, les significations suivantes :
- Y, si A est un groupe A¹, ne représente rien ou représente un chaînon CH₂ ou O, et si A est un groupe A², représente un chaînon CH₂ ou O ;
- l'indice n, si Y ne représente rien, vaut 0, 1, 2 ou 3, mais vaut 1 si Y représente un chaînon CH₂ ou O ;
- l'indice m, si Y ne représente rien, vaut 0, 1, 2 ou 3, mais vaut 1 si Y représente un chaînon CH₂ ou O ;
- l'indice r vaut 1, 2, 3 ou 4 ;
- R¹ représente un atome d'hydrogène, un groupe alkyle en C₁₋₁₀, aryle en C₆₋₁₂, aryl-alkyle en C₁₋₁₀ ou bicyclique en C₅₋₁₂, ou un groupe symbolisé par -R¹⁰-X- ;
- R² représente un groupe hydrocarboné aliphatique en C₁₋₂₀ éventuellement interrompu par un atome d'oxygène ou de soufre, cycloaliphatique en C₄₋₁₂, bicyclique en C₅₋₁₂, aryle en C₆₋₁₄ ou alkyl-aryle en C₇₋₂₀, lequel groupe porte, dans chaque cas, r substituant(s) représentés par ce qui est entre crochets ;
- R³ représente un groupe symbolisé par -CO-OR⁹, -CO-R⁹, -S(O)R⁹, -SO₂R⁹, -SO₂(OR⁹), -PO(OR⁹)₂, -R⁹ ou -CN, ou un atome d'hydrogène ;
- R⁴ représente un groupe symbolisé par -CO-OR⁹, -CO-R⁹, -S(O)R⁹, -SO₂R⁹, -SO₂(OR⁹), -PO(OR⁹)₂, -CN ou -R¹¹-R¹⁰-X- ;
- les symboles R⁵ à R⁸, indépendamment les uns des autres, représentent un atome d'hydrogène, un groupe symbolisé par -CO-OR⁹, -CO-NHR⁹, -CO-NR⁹₂, -CO-R⁹ ou -CN, un groupe alkyle en C₁₋₂₀ éventuellement interrompu par un atome d'oxygène ou de soufre, ou un groupe cycloaliphatique en C₄₋₁₂, bicyclique en C₅₋₁₂, aryle en C₆₋₁₄ ou alkyl-aryle en C₇₋₂₀, ou bien au moins deux de ces groupes constituent, conjointement avec l'atome de carbone auquel ils sont liés, un système cyclique comportant de 5 à 8 chaînons ;
- R⁹ représente un groupe alkyle en C₁₋₂₀ éventuellement interrompu par un atome d'oxygène ou de soufre, ou un groupe cycloaliphatique en C₄₋₁₂, bicyclique en C₅₋₁₂, aryle en C₆₋₁₄ ou alkyl-aryle en C₇₋₂₀ ;
- R¹⁰ ne représente rien ou représente un groupe alcanediyle en C₁₋₂₀ éventuellement interrompu par un atome d'oxygène ou de soufre, ou un groupe cycloaliphatique en C₄₋₁₂, bicyclique en C₅₋₁₂, arènediyle en C₆₋₁₄ ou alcanediyl-arènediyle en C₇₋₂₀ ;
- R¹¹ représente un raccord symbolisé par -COO-, -CO-, -SO-, -SO₂-, -S(O₂)O- ou -P(O)(OR⁹)O- ;
- et X ne représente rien ou représente un raccord symbolisé par -O-CO-, -CO-O-, -NH-CO-, -CO-NH-, -NH-CO-O- ou -O-CO-NH-, étant entendu que, si R¹⁰ ne représente rien, X non plus ne représente rien ;
étant entendu que :
- les groupes représentés par R² et R⁵ à R⁹ peuvent porter un substituant ou des substituants, ou n'en porter aucun ;
- soit R⁴ représente un groupe symbolisé par -R¹¹-R¹⁰-X-, soit R¹ représente un groupe symbolisé par -R¹⁰-X-, et le substituant représenté par ce qui est entre crochets est lié au groupe R² par l'intermédiaire du raccord X ;
- plusieurs groupes d'un même type peuvent être identiques ou différents ;
- et les substituants éventuellement portés par les groupes R² et R⁵ à R⁹ sont choisis parmi les groupes alkyle, les atomes d'halogène, les substituants méthoxy, éthoxy, vinyle, acrylyle et méthacrylyle, les groupes symbolisés par COOR¹², SiCl₃ ou Si(OR¹³)₃, et les groupes mésogènes, étant entendu que :
- R¹² représente un atome d'hydrogène ou un groupe alkyle en C₁₋₁₀, aryle en C₆₋₁₂, aryl-alkyle en C₆₋₁₀ ou bicyclique en C₅₋₁₂ ;
- et R¹³ représente un atome d'hydrogène ou un groupe alkyle en C₁₋₁₀ ;
ou un stéréoisomère d'un tel composé, ou un mélange de tels composés ;
b) et un amorceur de polymérisation radicalaire.

2. Composition conforme à la revendication 1, dans laquelle A est un groupe A¹, R¹ représente un groupe symbolisé par -R¹⁰-X-, et Y ne représente rien.

3. Composition conforme à la revendication 1, dans laquelle A est un groupe A¹, R⁴ représente un groupe symbolisé par -R¹¹-R¹⁰-X-, et Y ne représente rien.

4. Composition conforme à la revendication 1, dans laquelle A est un groupe A², R¹ représente un groupe symbolisé par -R¹⁰-X-, et Y représente un chaînon CH₂ ou O.

5. Composition conforme à l'une des revendications 1 à 4, dans laquelle au moins l'un des symboles de la formule (1) a l'une des significations suivantes :
- Y, si A est un groupe A¹, ne représente rien, et si A est un groupe A², représente un chaînon CH₂ ou O ;
- l'indice n vaut 1 ;
- l'indice m vaut 1 ;
- l'indice r vaut 1 ou 2 ;
- R¹ représente un atome d'hydrogène, un groupe alkyle en C₁₋₅, un groupe bicyclique en C₅₋₁₂, ou en particulier un groupe symbolisé par -R¹⁰-X- ;
- R² représente un groupe hydrocarboné aliphatique en C₁₋₆ éventuellement interrompu par un atome d'oxygène, ou un groupe cycloaliphatique en C₆₋₈, bicyclique en C₆₋₈, aryle en C₆₋₁₀ ou alkyl-aryle en C₇₋₁₀ ;
- R³ représente un groupe symbolisé par -CO-OR⁹, -CO-R⁹, -SO₂R⁹, -R⁹ ou -CN, ou un atome d'hydrogène ;
- R⁴ représente un groupe symbolisé par -CO-OR⁹, -CO-R⁹, -SO₂R⁹, -CN ou -R¹¹-R¹⁰-X- ;
- les symboles R⁵ à R⁸, indépendamment les uns des autres, représentent un atome d'hydrogène, un groupe symbolisé par -CO-OR⁹, -CO-R⁹ ou
- CN, un groupe alkyle en C₁₋₆ éventuellement interrompu par un atome d'oxygène, ou un groupe cycloaliphatique en C₆₋₈, bicyclique en C₆₋₈, aryle en C₆₋₁₀ ou alkyl-aryle en C₇₋₁₀ ;
- R⁹ représente un groupe alkyle en C₁₋₆ éventuellement interrompu par un atome d'oxygène, ou un groupe cycloaliphatique en C₆₋₈, bicyclique en C₆₋₈, aryle en C₆₋₁₀ ou alkyl-aryle en C₇₋₁₀, et en particulier un groupe alkyle en C₁₋₃ ;
- R¹⁰ ne représente rien ou représente un groupe alcanediyle en C₁₋₁₀ éventuellement interrompu par un atome d'oxygène, un groupe bicyclique en C₆₋₉ ou un groupe alcanediyl-arènediyle en C₇₋₁₀, et en particulier, ne représente rien ou représente un groupe alcanediyle en C₁₋₆ ;
- R¹¹ représente un raccord symbolisé par -COO-, -CO- ou -SO₂-, et en particulier -COO- ;
- et X ne représente rien ou représente un raccord symbolisé par -O-CO-ou -CO-O-.

6. Composition conforme à l'une des revendications 1 à 5, qui contient en plus au moins un autre monomère polymérisable par voie radicalaire.

7. Composition conforme à la revendication 6, qui contient, en tant que monomère supplémentaire polymérisable par voie radicalaire, un monomère polyfonctionnel.

8. Composition conforme à la revendication 7, qui contient, en tant que monomère polyfonctionnel polymérisable par voie radicalaire, un acrylate ou méthacrylate difonctionnel ou polyfonctionnel, comme les di(méth)acrylate de bisphénol A, bis-GMA (produit d'addition obtenu à partir d'acide méthacrylique et d'éther diglycidylique de bisphénol A), UDMA (produit d'addition obtenu à partir de méthacrylate de 2-hydroxyéthyle et de 2,2,4-triméthyl-hexaméthylène-diisocyanate), di(méth)acrylate de diéthylène-glycol, triéthylèneglycol ou tétraéthylèneglycol, tri(méth)-acrylate de triméthylolpropane, tétra(méth)acrylate de pentaérythritol, di(méth)acrylate de butanediol, di(méth)acrylate de 1,10-décanediol, et di(méth)acrylate de 1,12-dodécanediol, un uréthane obtenu à partir d'acide 2-(hydroxyméthyl)-acrylique et d'un diisocyanate comme le 2,2,4-triméthyl-hexaméthylène-diisocyanate ou l'isophorone-diisocyanate, une pyrrolidone réticulable comme le 1,6-bis(3-vinyl-pyrrolidone-2-yl)-hexane, ou un bis-acrylamide ou bis-(méth)acrylamide comme les méthylène-bis(acrylamide); éthylène-bis(acrylamide), N,N'-diéthyl-1,3-bis(acrylamido)-propane, 1,3-bis(méthacrylamido)-propane, 1,4-bis(acrylamido)-bùtane et N,N'-bis(acryloyl)-pipérazine, ou un mélange de deux de ces monomères ou plus.

9. Composition conforme à l'une des revendications 1 à 8, qui contient en outre une charge.

10. Composition conforme à l'une des revendications 1 à 9, qui contient :
a) 1 à 80 % en poids d'un cyclopropyl-acrylate de formule (1),
b) 0,01 à 5 % en poids d'un amorceur de polymérisation radicalaire,
c) 0 à 60 % en poids d'un monomère polymérisable par voie radicalaire,
d) 0 à 40 % en poids d'un solvant,
e) et 0 à 20 % en poids d'une charge.

11. Composition conforme à l'une des revendications 1 à 9, qui contient :
a) 1 à 60 % en poids d'un cyclopropyl-acrylate de formule (1),
b) 0,01 à 5 % en poids d'un amorceur de polymérisation radicalaire,
c) 0 à 60 % en poids d'un monomère polymérisable par voie radicalaire,
d) et 20 à 60 % en poids d'une charge.

12. Composition conforme à l'une des revendications 1 à 9, qui contient :
a) 1 à 45 % en poids d'un cyclopropyl-acrylate de formule (1),
b) 0,01 à 5 % en poids d'un amorceur de polymérisation radicalaire,
c) 0 à 50 % en poids d'un monomère polymérisable par voie radicalaire,
d) et 30 à 85 % en poids d'une charge.

13. Composition conforme à l'une des revendications 1 à 9, qui contient :
a) 1 à 95 % en poids d'un cyclopropyl-acrylate de formule (1),
b) 0,01 à 5 % en poids d'un amorceur de polymérisation radicalaire,
c) 0 à 60 % en poids d'un monomère polymérisable par voie radicalaire,
d) et 0 à 20 % en poids d'une charge.

14. Composition conforme à l'une des revendications 1 à 13, qui contient en outre au moins un autre composant choisi dans l'ensemble formé par les stabilisants, les absorbeurs UV, les colorants, les pigments et les lubrifiants.

15. Composition conforme à l'une des revendications 1 à 14, sous forme durcie.

16. Utilisation d'un cyclopropyl-acrylate de formule (1) en vue de la fabrication d'un matériau dentaire.

17. Utilisation d'une composition conforme à l'une des revendications 1 à 15 en tant que matériau dentaire.

18. Utilisation d'un cyclopropyl-acrylate ou d'une composition, conforme à la revendication 16 ou 17, dans laquelle le matériau dentaire est un adhésif.

19. Utilisation d'un cyclopropyl-acrylate ou d'une composition, conforme à la revendication 16 ou 17, dans laquelle le matériau dentaire est un ciment.

20. Utilisation d'un cyclopropyl-acrylate ou d'une composition, conforme à la revendication 16 ou 17, dans laquelle le matériau dentaire est un matériau de remplissage.

21. Utilisation d'un cyclopropyl-acrylate ou d'une composition, conforme à la revendication 16 ou 17, dans laquelle le matériau dentaire est un matériau de revêtement.
